# EUROPEAN PATENT APPLICATION

(11) **EP 2 738 174 A2**
(43) Date of publication of application: **04.06.2014**
(21) Application number: 12817633.6
(22) Date of filing: 29.06.2012
(51) Int. Cl.: C07D 495/04, C07D 487/04, A61K 31/519, A61P 3/10

(54) **NOVEL PYRIMIDINE DERIVATIVE AND PHARMACEUTICAL COMPOSITION INCLUDING SAME AS AN ACTIVE INGREDIENT**

(30) Priority: 27.07.2011 KR 20110074706
(71) Applicant: Hanmi Pharmaceutical Co., Ltd., Gyeonggi-do 445-813 (KR)
(72) Inventor: PARK, Chul Hyun, Seongnam-si Gyeonggi-do 463-010 (KR); KIM, Won Jeoung, Suwon-si Gyeonggi-do 440-716 (KR); JUNG, Young Hee, Seoul 131-856 (KR); KIM, Nam Du, Hwaseong-si Gyeonggi-do 445-755 (KR); CHANG, Young Kil, Seoul 138-220 (KR); KIM, Maeng Sup, Seoul 134-060 (KR)
(74) Representative: Goddar, Heinz J.
(86) International application number: PCT/KR2012/005193
(87) International publication number: WO 2013/015535

(57) **Abstract**

The present invention relates to a compound represented by formula (I) for inhibiting the activity of diacylglycerol O-acyltransferase type 1 (DGAT1), and pharmaceutically acceptable salts thereof, and a pharmaceutical composition comprising same as an active ingredient. The compound of the present invention may be used effectively in the treatment or prevention of a disease or condition mediated by the activity of DGAT1 such as obesity, type II diabetes, dyslipidemia, metabolic syndrome, and the like, without any adverse effects: 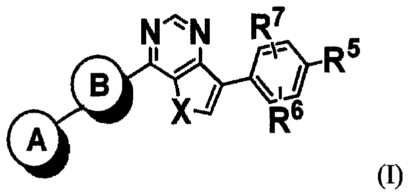 wherein A, B, X, and R⁵ to R⁷ are the same as defined in the specification.

## Description

### FIELD OF THE INVENTION

The present invention relates to a compound selected from the group consisting of a novel pyrimidine derivative or pharmaceutically acceptable salts thereof for inhibiting the activity of diacylglycerol O-acyltransferase type 1 (DGAT1), and a pharmaceutical composition comprising same as an active ingredient.

### BACKGROUND OF THE INVENTION

Triglycerides or triacylglycerols represent the major form of stored energy in eukaryotes. In mammals, synthesis of such compounds mostly takes place in the small intestine, liver and adipose cells. The primary role of triglycerides or triacylglycerols includes dietary fat absorption, packaging of newly synthesized fatty acids, and storage in fat tissue *(see* Subauste and Burant, Endocrine & Metabolic Disorders (2003) 3, 263-270).

Diacylglycerol O-acyltransferase (DGAT), also known as diglyceride acyltransferase, is a key enzyme in triglycerides synthesis. DGAT catalyzes the final and rate-limiting step in triacylglycerol synthesis from 1,2-diacylglycerol (DAG) and long chain fatty acyl CoA as substrates. Therefore, DGAT plays an essential role in the metabolism of cellular DAG, and is very important for triglyceride production and energy storage homeostasis *(see* Mayorek et al., European Journal of Biochemistry (1989), 182, 395-400).

An excessive bioaccumulation caused by imbalances in triglyceride metabolism, both absorption and de novo synthesis, may lead to pathogenesis of a variety of diseases. Such diseases include obesity, insulin resistance syndrome, type II diabetes, dyslipidemia, metabolic syndrome (syndrome X) and coronary heart disease *(see* Kahn, Nature Genetics (2000) 25, 6-7; Yanovski, New England Journal of Medicine (2002) 346, 591-602); Lewis et al., Endocrine Reviews (2002)23, 201; Brazil, Nature Reviews Drug Discovery (2002) 1, 408; Malloy and Kane, Advances in Internal Medicine (2001) 47, 111; Subauste and Burant, Endocrine & Metabolic Disorders (2003) 3, 263-270; and Yu and Ginsberg, Annals of Medicine (2004) 6, 252-261). Accordingly, compounds capable of decreasing the synthesis of triglycerides from DAG by inhibiting or lowering the activity of the DGAT enzyme would be of value as therapeutic agents for the treatment diseases associated with abnormal metabolism of triglycerides.

Known inhibitors of DGAT include: dibenzoxazepinones *(see* Ramharack et al., EP 1 219 716 and Burrows et al., 26th National Medicinal Chemistry Symposium (1998) poster C-22), substituted amino-pyrimidino-oxazines *(see* Fox et al., WO 2004/047755), chalcones such as xanthohumol *(see* Tabata et al., Phytochemistry (1997) 46, 683-687; and Casaschi et al., Journal of Nutrition (2004) 134, 1340-1346), substituted benzyl-phosphonates *(see* Kurogi et al., Journal of Medicinal Chemistry (1996) 39, 1433-1437; Goto et al., Chemistry and Pharmaceutical Bulletin (1996) 44, 547-551; Ikeda et al., Thirteenth International Symposium on Athersclerosis (2003), abstract 2P-0401; and Miyata et al., JP 2004-067635), aryl alkyl acid derivatives *(see* Smith et al., WO 2004/100881 and US 2004/0224997), furan and thiophene derivatives (*see* WO 2004/022551), pyrrolo[1,2b]pyridazine derivatives *(see* Fox et al., WO 2005/103907), and substituted sulfonamide *(see* Budd Haeberlein and Buckett, WO 2005/044250).

Also known DGAT inhibitors are: 2-bromo-palmitic acid *(see* Colman et al., Biochimica et Biophysica Acta (1992) 1125, 203-209), 2-bromo-octanoic acid *(see* Mayorek and Bar-Tana, Journal of Biological Chemistry (1985) 260, 6528-6532), roselipins *(see* Noriko et al., Journal of Antibiotics (1999) 52, 815-826), amidepsin *(see* Tomoda et al., Journal of Antibiotics (1995) 48, 942-7), isochromophilone, prenylflavonoids *(see* Chung et al., Planta Medica (2004) 70, 258-260), polyacetylenes (*see* Lee et al, Planta Medica (2004) 70, 197-200), cochlioquinones *(see* Lee et al., Journal of Antibiotics (2003) 56, 967-969), tanshinones *(see* Ko et al., Archives of Pharmaceutical Research (2002) 25, 446-448), gemfibrozil *(see* Zhu et al., Atherosclerosis (2002) 164, 221-228), and substituted quinolones (*see* Ko et al., Planta Medica (2002) 68, 1131-1133). Also, they are known as modulators of DGAT activity *(see* Monia and Graham, US 2004/0185559).

Aforementioned inhibitors of DGAT are known to have efficacy in the treatment of obesity, insulin resistance syndrome, type II diabetes, dyslipidemia, metabolic syndrome and coronary heart disease *(see* Fox et al., WO 2004/04755). However, there still remains a need in the art for additional DGAT inhibitors having IC₅₀ values less than about 1 µM and efficacy in the treatment of metabolic disorders such as obesity, type II diabetes and metabolic syndrome.

### SUMMARY OF THE INVENTION

Therefore, it is an object of the present invention to provide a novel pyrimidine derivative for inhibiting the activity of diacylglycerol O-acyltransferase type 1 or pharmaceutically acceptable salts thereof.

It is another object of the present invention to provide a pharmaceutical composition comprising same as an active ingredient.

In accordance with one aspect of the present invention, there is provided a novel pyrimidine derivative for inhibiting the activity of diacylglycerol O-acyltransferase type 1 or pharmaceutically acceptable salts thereof.

In accordance with another aspect of the present invention, there is provided a pharmaceutical composition comprising same as an active ingredient.

The present invention is explained in detail hereinafter.

The present invention provides a compound selected from the group consisting of the compound of formula (I) and pharmaceutically acceptable salts thereof: 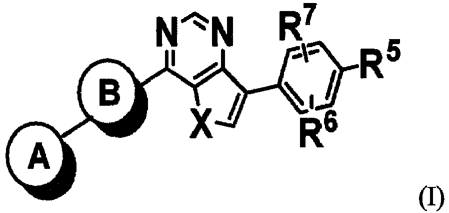
wherein X is each independently S or NR wherein R is H or C₁₋₄alkyl;
A is 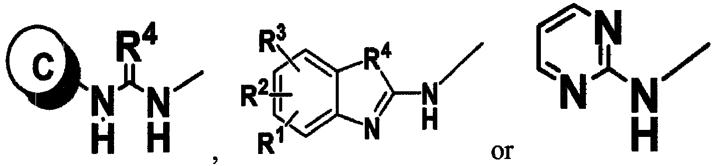 wherein C is hydrogen, C₁₋₆alkyl, C₃₋₈cycloalkyl, C₆₋₁₄aryl, C₅₋₁₃heteroaryl or C₃₁₃heterocycloalkyl, wherein the heteroaryl or heterocycloalkyl is unsubstituted or substituted with C₁₋₆alkyl, halogen, diC₁₋₆alkylamino or C₁₋₆alkoxy, and the aryl is unsubstituted or substituted with halogen, C₁₋₆alkyl, halogen-substituted C₁₋₆alkyl, C₁₋₄alkoxy, C₃₋₈cycloalkyl, C₁₋₆alkylamino, di₁₋₆alkylamino, C₆₁₄aryl, C₅₋₁₃heteroaryl or C₃₋₁₃heterocycloalkyl (wherein the aryl, heteroaryl or heterocycloalkyl is unsubstituted or substituted with C₁₋₆alkyl, halogen, diC₁₆alkylamino or C₁₋₆alkoxy),
R⁴ is O, S or NR wherein R is H or C₁₋₄alkyl;
B is unsubstituted or substituted C₆₋₁₄aryl or heteroaryl;
R⁵ is C₁₋₆alkyl unsubstituted or substituted with a substituent selected from the group consisting of carboxy, C₁₋₄alkoxycarbonyl, aminocarboxyl and hydroxyl; C₃₋₈cycloalkyl unsubstituted or substituted with carboxy or C₁₄alkoxycarbonyl; or carboxyC₁₋₆alkylamido, C₁₋₆alkylsulfonyl, C₁₋₆alkylamino, C₁₋₆alkylamido, di₁₋₆alkylamino, C₁₋₆alkoxy, C₁₋₆alkylcarbonyl, C₁₆alkoxycarbonyl, C₆₋₁₄aryl, C₅₋₁₃heteroaryl or C₃₋₁₃heterocycloalkyl wherein the aryl, heteroaryl or heterocycloalkyl is unsubstituted or substituted with C₁₋₆alkyl, halogen, diC₁₋₆alkylamino or C₁₋₆alkoxy; and
R⁶ and R⁷ are each independently H, halogen, C₁₋₄alkoxy unsubstituted or substituted with 1 to 3 halogens, C₃₋₈cycloalkyl, C₁₋₆alkylamino, diC₁₆alkylamino, C₆₋₁₄aryl, C₅₋₁₃heteroaryl or C₃₋₁₃heterocycloalkyl, .

In the compound of formula (I) of the present invention, preferably,
X is each independently S or NR wherein R is H or C₁₋₄alkyl;
A is 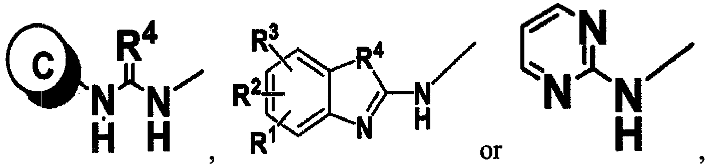 wherein C is C₃₋₈cycloalkyl, C₆₋₁₄aryl, C₅₋₁₃heteroaryl or C₃₁₃heterocycloalkyl, wherein the heteroaryl or heterocycloalkyl is unsubstituted or substituted with halogen, and the aryl unsubstituted or substituted with halogen, C₁₋₆alkyl, C₁₋₆alkyl substituted with halogen, C₁₋₄alkoxy, C₁₆alkylamino, diC₁₋₆alkylamino, C₅₋₁₃heteroaryl or C₃₋₁₃heterocycloalkyl,
R⁴ is O or S;
B is unsubstituted or substituted C₆₋₁₄aryl;
R⁵ is C₁₋₆alkyl substituted with a substituent selected from the group consisting of carboxy, C₁₋₄alkoxycarbonyl, aminocarboxyl and hydroxyl, carboxyC₃₋₈cycloalkyl, carboxyC₁₋₆alkylamido or C₁₋₆alkylsulfonyl; and
R⁶ and R⁷ are each independently H or halogen.

As used herein, the term "halo" refers to fluoro, bromo, chloro or iodo.

As used herein, the term "alkyl" refers to straight or branched saturated chain radicals containing 1 to 6 carbon atoms. Particular examples thereof include, but not limited to, methyl, ethyl, *n*-propyl, isopropyl, *n*-butyl, isobutyl, *t*-butyl, *n*-pentyl, isopentyl, hexyl and the like.

As used herein, the term "alkoxy" refers to an -ORₐ group, wherein Rₐ is alkyl as defined above. Particular examples thereof include, but not limited to, methoxy, ethoxy, *n*-propoxy, isopropoxy, *n*-butoxy, *n*-butoxy and the like.

As used herein, the term "heteroaryl" refers, unless otherwise specified, to a mono- or bicyclic aromatic heterocyclic ring which comprises at least one heteroatom selected from O, N and S. Examples of a monocyclic heterocycle include, but not limited to, thiazolyl, oxazolyl, thiophenyl, furanyl, pyrrolyl, imidazolyl, isooxazolyl, pyrazolyl, triazolyl, thiadiazolyl, tetrazolyl, oxadiazolyl, pyridinyl, pyridazinyl, pyrimidinyl, pyrazinyl and similar groups thereof. Examples of a bicyclic heterocycle include, but not limited to, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, furinyl, furopyridinyl and similar groups thereof.

As used herein, the term "heterocycloalkyl" refers, unless otherwise specified, to a cycloalkyl ring which contains a heteroatom selected from O, N and S.

More preferred examples of the compound of the formula (I) in accordance with the present invention include:
1) 2-(4-(4-(4-(3-phenylureido)phenyl)thieno[3,2-*d*]pyrimidin-7-yl)phenyl)acetic acid;
2) 2-(4-(4-(4-(3-(3-chlorophenyl)ureido)phenyl)thieno[3,2-*d*]pyrimidin-7-yl) phenyl)acetic acid;
3) 4-(4-(4-(3-(3-chloraphenyl)ureido)phenyl)thieno[3,2-*d*]pyrimidin-7-yl)benzoic acid;
4) 2-(4-(4-(4-(3-(3,4-difluorophenyl)ureido)phenyl)thieno[3,2-*d*]pyrimidin-7-yl)phenyl)acetic acid;
5) 2-(4-(4-(4-(3-(2-(trifluoromethoxy)phenyl)ureido)phenyl)thieno[3,2-*d*]pyrimidin-7-yl)phenyl)acetic acid;
6) 2-(4-(4-(3-(3-chlorophenyl)ureido)thieno[3,2-*d*]pyrimidin-7-yl)benzoamido)acetic acid;
7) 2-(4-(4-(4-(3-(4-methoxyphenyl)ureido)phenyl)thieno[3,2-d]pyrimidin-7-yl)phenyl)acetic acid;
8) (S)-2-(4-(4-(3-(3-chlorophenyl)ureido)thieno[3,2-*d*]pyrimidin-7-yl)benzoamido)-3-methylbutanoic acid;
9) 3-(4-(4-(4-(3-(3-chlorophenyl)ureido)phenyl)thieno[3,2-*d*]pyrimidin-7-yl)phenyl)propanoic acid;
10) 2-(4-(4-(4-(3-ethylureido)phenyl)thieno[3,2-*d*]pyrimidin-7-yl)phenyl)acetic acid;
11) methyl 2-(4-(4-(4-(3-(3-chlorophenyl)ureido)phenyl)thieno[3,2-*d*]pyrimidin-7-yl)phenyl)acetate;
12) 2-(4-(4-(4-(3-(2-chlorophenyl)ureido)phenyl)thieno[3,2-*d*]pyrimidin-7-yl)phenyl)acetic acid;
13) 2-(4-(4-(4-(3-(4-chlorophenyl)ureido)phenyl)thieno[3,2-*d*]pyrimidin-7-yl)phenyl)acetic acid;
14) 2-(4-(4-(4-(benzo[*d*]thiazol-2-ylamino)phenyl)thieno[3,2-d]pyrimidin-7-yl)phenyl)acetic acid;
15) 2-(4-(4-(4-(pyrimidin-2-ylamino)phenyl)thieno[3,2-*d*]pyrimidin-7-yl)phenyl)acetic acid;
16) 2-(4-(4-(4-(3-pyridin-3-ylureido)phenyl)thieno[3,2-*d*]pyrimidin-7-yl)phenyl)acetic acid;
17) 2-(4-(4-(4-(6-chlorobenzo[*d*]thiazol-2-ylamino)phenyl)thieno[3,2-*d*]pyrimidin-7-yl)phenyl)acetic acid;
18) 2-(4-(4-(4-(3-(2-chloropyridin-4-yl)ureido)phenyl)thieno[3,2-d]pyrimidin-7-yl)phenyl)acetic acid;
19) 2-(4-(4-(4-(3-(4-(dimethylamino)phenyl)ureido)phenyl)thieno[3,2-*d*]pyrimidin-7-yl)phenyl)acetic acid;
20) 2-(4-(4-(4-(3-(4-chloropyridin-2-yl)ureido)phenyl)thieno[3,2-*d*]pyrimidin-7-yl)phenyl)acetic acid;
21) 2-(4-(4-(4-(3-pyridin-2-ylureido)phenyl)thieno[3,2-*d*]pyrimidin-7-yl)phenyl)acetic acid;
22) 2-(4-(4-(4-(3-pyridin-4-ylureido)phenyl)thieno[3,2-*d*]pyrimidin-7-yl)phenyl)acetic acid;
23) 4-(4-(4-(4-(3-(3-chlorophenyl)ureido)phenyl)thieno[3,2-*d*]pyrimidin-7-yl)phenyl)butanoic acid;
24) 3-(4-(4-(4-(3-(2-chlorophenyl)ureido)phenyl)thieno[3,2-*d*]pyrimidin-7-yl)phenyl)propanoic acid;
25) 3-(4-(4-(4-(3-(3,4-difluorophenyl)ureido)phenyl)thieno[3,2-*d*]pyrimidin-7-yl)phenyl)propanoic acid;
26) 3-(4-(4-(4-(3-(4-methoxyphenyl)ureido)phenyl)thieno[3,2-*d*]pyrimidin-7-yl)phenyl)propanoic acid;
27) 3-(4-(4-(4-(3-(3-fluorophenyl)ureido)phenyl)thieno[3,2-*d*]pyrimidin-7-yl)phenyl)propanoic acid;
28) 3-(4-(4-(4-(3-(4-(dimethylamino)phenyl)ureido)phenyl)thieno[3,2-*d*]pyrimidin-7-yl)phenyl)propanoic acid;
29) 3-(4-(4-(4-(3-(2,3-difluorophenyl)ureido)phenyl)thieno[3,2-*d*]pyrimidin-7-yl)phenyl)propanoic acid;
30) 3-(4-(4-(4-(3-(4-chloropyridin-2-yl)ureido)phenyl)thieno[3,2-*d*]pyrimidin-7-yl)phenyl)propanoic acid;
31) 2-(4-(4-(4-(3-cyclohexylureido)phenyl)thieno[3,2-*d*]pyrimidin-7-yl)phenyl)acetic acid;
32) 2-(4-(4-(4-(3-pyridazin-3-ylureido)phenyl)thieno[3,2-*d*]pyrimidin-7-yl)phenyl)acetic acid;
33) 3-(4-(4-(4-(3-(2-chloro-6-methylphenyl)ureido)phenyl)thieno[3,2-d]pyrimidin-7-yl)phenyl)propanoic acid;
34) 3-(4-(4-(4-(3-(3-chloro-4-methoxyphenyl)ureido)phenyl)thieno[3,2-*d*]pyrimidin-7-yl)phenyl)propanoic acid;
35) 1-(4-(4-(4-(3-(3-chlorophenyl)ureido)phenyl)thieno[3,2-*d*]pyrimidin-7-yl)phenyl)cyclopropancarboxylic acid;
36) 2-(4-(4-(4-(3-(3-chlorophenyl)ureido)phenyl)thieno[3,2-*d*]pyrimidin-7-yl)phenyl)-2-methylpropanoic acid;
37) 3-(4-(4-(4-(3-(3-methoxyphenyl)ureido)phenyl)thieno[3,2-*d*]pyrimidin-7-yl)phenyl)propanoic acid;
38) 3-(4-(4-(4-(3-(2-methoxyphenyl)ureido)phenyl)thieno[3,2-*d*]pyrimidin-7-yl)phenyl)propanoic acid;
39) 3-(4-(4-(4-(3-p-tolylureido)phenyl)thieno[3,2-*d*]pyrimidin-7-yl)phenyl)propanoic acid;
40) 3-(4-(4-(4-(3-(5-fluoro-2-methylphenyl)ureido)phenyl)thieno[3,2-*d*]pyrimidin-7-yl)phenyl)propanoic acid;
41) 3-(4-(4-(4-(3-(2-fluoro-5-(trifluoromethyl)phenyl)ureido)phenyl) thieno[3,2-*d*]pyrimidin-7-yl)phenyl)propanoic acid;
42) 3-(4-(4-(4-(3-(3,4,5-trimethoxyphenyl)ureido)phenyl)thieno[3,2-*d*]pyrimidin-7-yl)phenyl)propanoic acid;
43) 3-(4-(4-(4-(3-(2,3-dichlorophenyl)ureido)phenyl)thieno[3,2-*d*]pyrimidin-7-yl)phenyl)propanoic acid;
44) 3-(4-(4-(4-(3-(3-(trifluoromethyl)phenyl)ureido)phenyl)thieno[3,2-*d*]pyrimidin-7-yl)phenyl)propanoic acid;
45) 3-(4-(4-(4-(3-(2,5-difluorophenyl)ureido)phenyl)thieno[3,2-*d*]pyrimidin-7-yl)phenyl)propanoic acid;
46) 3-(4-(4-(4-(3-(4-morpholinophenyl)ureido)phenyl)thieno[3,2-*d*]pyrimidin-7-yl)phenyl)propanoic acid;
47) 3-(4-(4-(4-(3-(2,3,5-trifluorophenyl)ureido)phenyl)thieno[3,2-*d*]pyrimidin-7-yl)phenyl)propanoic acid;
48) 3-(4-(4-(4-(3-(2-chloro-5-methoxyphenyl)ureido)phenyl)thieno[3,2-*d*]pyrimidin-7-yl)phenyl)propanoic acid;
49) 3-(4-(4-(4-(3-(2-fluorophenyl)ureido)phenyl)thieno[3,2-*d*]pyrimidin-7-yl)phenyl)propanoic acid;
50) 3-(4-(4-(4-(3-cyclohexylureido)phenyl)thieno[3,2-*d*]pyrimidin-7-yl)phenyl)propanoic acid;
51) 3-(4-(4-(4-(3-(2-chlorophenyl)ureido)phenyl)-5-methyl-5*H-*pyrrolo[3,2-*d*]pyrimidin-7-yl)phenyl)propanoic acid;
52) 3-(4-(4-(4-(3-(4-(1*H*-pyrrolo-1-yl)phenyl)ureido)phenyl)thieno[3,2-*d*]pyrimidin-7-yl)phenyl)propanoic acid;
53) 3-(4-(4-(4-(3-(4-(2*H*-tetrazol-5-yl)phenyl)ureido)phenyl)thieno[3,2-d]pyrimidin-7-yl)phenyl)propanoic acid;
54) 3-(4-(4-(4-(3-(4-(4-methylpiperazin-1-yl)phenyl)ureido)phenyl) thieno[3,2-*d*]pyrimidin-7-yl)phenyl)propanoic acid;
55) 3-(4-(4-(4-(3-(1-methylpiperidin-4-yl)ureido)phenyl)thieno[3,2-*d*]pyrimidin-7-yl)phenyl)propanoic acid;
56) 3-(4-(4-(4-(3-(4-(pyrrolidin-1-yl)phenyl)ureido)phenyl)thieno[3,2-*d*]pyrimidin-7-yl)phenyl)propanoic acid;
57) 3-(4-(4-(4-(3-(3-chlorophenyl)ureido)phenyl)thieno[3,2-*d*]pyrimidin-7-yl)phenyl)propanamide;
58) 1-(3-chlorophenyl)-3-(4-(7-(4-(3-hydroxypropyl)phenyl)thieno[3,2-*d*]pyrimidin-4-yl)phenyl)urea;
59) 3-(4-(4-(4-(3-(4-(4*H*-1,2,4-triazol-4-yl)phenyl)ureido)phenyl)thieno [3,2-*d*]pyrimidin-7-yl)phenyl)propanoic acid;
60) 3-(4-(4-(4-(3-(4-(oxazol-2-yl)phenyl)ureido)phenyl)thieno[3,2-*d*]pyrimidin-7-yl)phenyl)propanoic acid;
61) 3-(4-(4-(3-(3-chlorophenyl)ureido)thieno[3,2-*d*]pyrimidin-7-yl)phenyl)propanoic acid;
62) 3-(4-(4-(4-(3-(2-fluoro-3-(trifluoromethyl)phenyl)ureido)phenyl) thieno[3,2-*d*]pyrimidin-7-yl)phenyl)propanoic acid;
63) 3-(4-(4-(4-(3-(4-fluoro-2-(trifluoromethyl)phenyl)ureido)phenyl) thieno[3,2-*d*]pyrimidin-7-yl)phenyl)propanoic acid;
64) 3-(4-(4-(4-(3-(3-chlorophenyl)ureido)phenyl)thieno[3,2-*d*]pyrimidin-7-yl)-2-fluorophenyl)propanoic acid; and
65) 1-(3-chlorophenyl)-3-(4-(7-(4-(methylsulfon)phenyl)thieno[3,2-*d*]pyrimidin-4-yl)phenyl)urea.

In one embodiment of the present invention, the compound of formula (I) of the present invention may be prepared by a method as shown in Reaction Scheme 1 by using the compound of formula (II) as an intermediate:

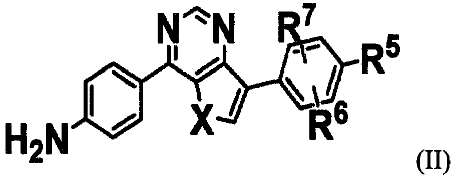

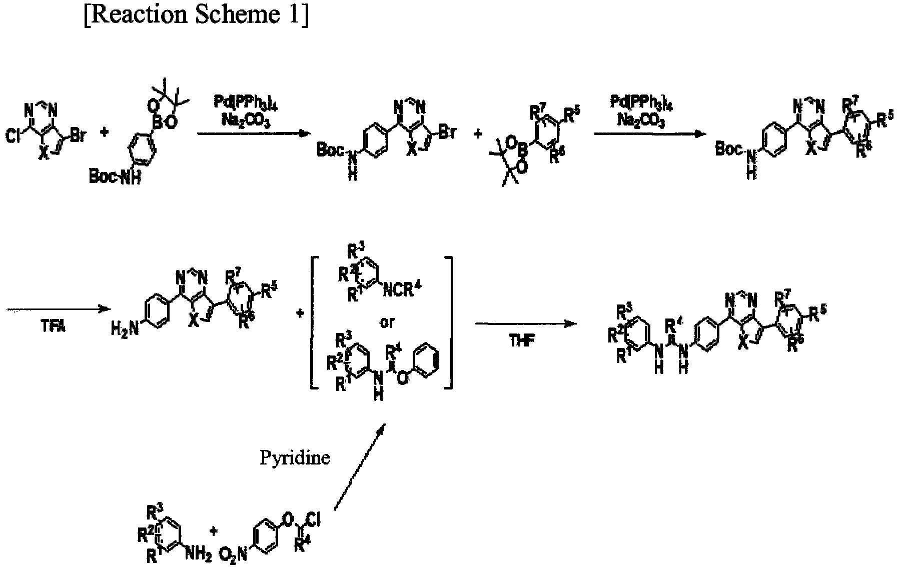

Specifically, 7-bromo-4-chlorothieno[3,2-*d*]pyrimidine or N-substituted 7-bromo-4-chloro-pyrrolo[3,2-*d*]pyrimidine is subjected to a Suzuki coupling reaction with *tert*-butyl 4-(4,4,5,5-tetramethyl-1,3,2-dioxaborolan-2-yl)phenylcarbamate to replace the chloride in position 4 of thieno[3,2-*d*]pyrimidine or pyrrolo[3,2-*d*]pyrimidine. Then, the Suzuki coupling reaction is repeated, followed by a deprotection reaction to yield the compound of formula (II). Subsequently, the compound of formula (II) thus obtained is allowed to react with isocyanate or thioisocyanate to obtain the compound of formula (I). Alternatively, an aniline compound is subjected to a reaction with substituted phenyl chloroformate or substituted phenyl thiochloroformate, and then the resulting compound is allowed to react with the compound of formula (II) to obtain the compound of the formula (I).

In accordance with the present invention, the compound of formula (I) includes pharmaceutically acceptable salts thereof.

Specifically, the compound of formula (I) of the present invention may be used in the form of a pharmaceutically acceptable salt derived from an inorganic or organic acid. For example, such a salt includes salts of inorganic acids such as hydrochloric acid, sulfuric acid, nitric acid, phosphoric acid, perchloric acid, bromic acid and the like; salts of organic acids such as formic acid, acetic acid, propanoic acid, oxalic acid, succinic acid, benzoic acid, citric acid, maleic acid, malonic acid, malic acid, tartaric acid, gluconic acid, lactic acid, gentisic acid, fumaric acid, lactobionic acid, salicylic acid, phthalic acid, embonic acid, aspartic acid, glutamic acid, acetylsalicylic acid (aspirin) and the like; salts of amino acids such as glycine, alanine, vanillin, isoleucine, serine, cystein, cystine, aspartate, glutamine, lysine, arginine, tyrosine, proline and the like; salts of sulfonic acids such as methanesulfonic acid, ethanesulfonic acid, benzenesulfonic acid, toluenesulfonic acid and the like; metallic salts such as sodium, potassium and the like; and ammonium salts and the like. Also, examples of organic bases which may be used in the preparation of an organic base addition salt include tris(hydroxymethyl)methylamine, dicyclohexylamine and the like.

These pharmaceutically acceptable salts of the compound of formula (I) may be prepared in accordance with conventional methods known in the art. For example, the pharmaceutically acceptable salts may be prepared by dissolving the compound of formula (I) in a water miscible solvent such as methanol, ethanol, acetone or 1,4-dioxane and then adding a free acid or base, followed by a crystallization.

The compound of the present invention or pharmaceutically acceptable salts thereof can selectively and effectively inhibit the activity of DGAT1, and thus, may be used for the prevention or treatment of a disease or condition mediated by the activity of DGAT1.

Examples of the disease or condition mediated by the activity of DGAT1 include, but not limited to, metabolic disorders such as obesity, diabetes, anorexia nervosa, addephagia, dyscrasia, syndrome X, insulin resistance syndrome, hypoglycemia, hyperglycemia, hyperuricemia, hyperinsulinemia, hypercholesteremia, hyperlipidemia, dyslipidemia, complex dyslipidemia, hypertriglyceridemia, pancreatitis, non-alcoholic fatty liver disease, cardiovascular disorders (for example, atherosclerosis, arteriosclerosis, acute heart failure, congestive heart failure, coronary artery disease, cardiomyopathy, myocardial infarction, angina, hypertension, hypotension, stroke, ischemia, ischemia reperfusion injury, aortic disease, restenosis and angiostenosis), neoplastic diseases (for example, solid tumors (e.g., breast cancer, lung cancer, colorectal cancer, stomach cancer, and gastrointestinal cancers such as esophageal cancer and pancreatic cancer, prostate cancer, renal cancer, liver cancer, bladder cancer, cervical cancer, uterine cancer, testicular cancer, ovarian cancer), skin cancer, melanoma, lymphoma and endothelial cancer), skin disorders (for example, acne) and the like.

The present invention provides a pharmaceutical composition comprising the compound of formula (I) or pharmaceutically acceptable salts thereof for the prevention or treatment of a disease or condition mediated by the activity of DGAT1. In accordance with the present invention, preferably, the disease or condition mediated by the activity of DGAT1 is selected from the group consisting of obesity, hyperlipidemia, hypertriglyceridemia, lipid metabolism disorders, insulin resistance syndrome, glucose intolerance, diabetes, diabetic complications, cataract, gestational diabetes, non-alcoholic fatty liver disease, polycystic ovary syndrome, arteriosclerosis, atherosclerosis, diabetic sclerosis, ischemic heart diseases, bulimia, hypertension, cerebrovascular disorders, coronary artery disease, fatty liver, respiratory abnormality, backache, gonarthrosis, gout and cholelithiasis.

The pharmaceutical composition of the present invention comprising a thieno and pyrrolo[3,4-*d*]pyrimidine derivative represented by formula (I) or pharmaceutically acceptable salts thereof as an active ingredient. Pharmaceutically acceptable carrier, additive, excipient and the like may be added, and the composition may be prepared in the form of oral formulations, e.g., tablets, capsules, lozenges, liquids and suspensions, or parenteral formulations in accordance with conventional methods known in the art.

A solid formulation for oral administration may be prepared by mixing at least one thieno and pyrrolo [3,4-*d*]pyrimidine derivative with at least one excipient, e.g., starch, calcium carbonate, sucrose, lactose or gelatin. Also, lubricants such as magnesium stearate or talc may be used in addition to excipients.

The liquid formulations for oral administration include suspensions, solutions, oils, syrups and the like, and various excipients, e.g., wetting agents, sweetening agents, fragrances, preservatives and the like, may be used in addition to diluents such as water, liquid paraffin, etc.

The parenteral formulations include sterile aqueous solutions, nonaqueous solvents, suspension solvents, oils, lyophilized formulations or suppository formulations. Examples of the non-aqueous solvents or suspension solvents include propylene glycol, polyethylene glycol, or vegetable oils such as olive oil, any suitable ester such as ethyl oleate. Examples of base materials employable for the suppository formulations include Witepsol, Macrogol, Tween 61, cacao butter, laurin fat, glycerol gelatin and the like.

Also, a daily dose of the pharmaceutical composition in accordance with the present invention should be determined in light of various relevant factors including age, body weight, sex of the subject to be treated, the dosage form, the severity of the disease or condition, and the like. The pharmaceutical composition of the present invention may be administered, based on a human adult with the body weight of 70 kg, in the range of 20 to 200 mg/day, preferably 50 to 100 mg/day, in single or divided daily doses.

The inventive compound may be used in the treatment of a disease or condition mediated by the activity of DGAT1 such as obesity, type II diabetes, dyslipidemia, metabolic syndrome, etc. without showing any adverse effects.

### EMBODIMENTS FOR CARRYING OUT THE INVENTION

Hereinafter, the present invention is described more specifically by the following examples, but these are provided only for illustration purposes, and the present invention is not limited thereto.

The compound of Preparation Example 1 was prepared by using the reaction scheme below:

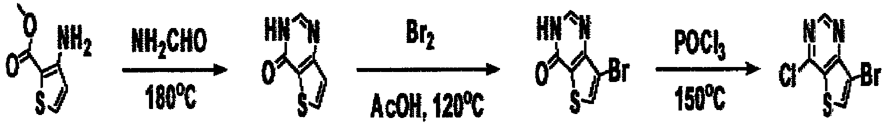

### Preparation Example 1: Preparation of 7-bromo-4-chlorothieno[3,2-d]pyrimidine

### Step 1: Preparation of 3H-thieno[3,2-d]pyrimidin-4-one

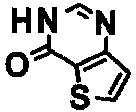

Methyl-3-aminothiothiophene-2-carboxylate (15 g, 98.6 mmol, Matrix, Cat. No. 018289, CAS [22288-78-4]) was dissolved in formamide (50 mL), and stirred for 5 hrs at 180°C. The resulting compound was further stirred for 2 hrs at room temperature. A solid thus obtained was filtered to obtain the title compound.
¹H NMR (300 MHz, DMSO-*d*₆): *δ* 12.48 (br, 1H), 8.18 (d, J = 5.1 Hz, 1H), 8.14 (s, 1H), 7.40 (d, J = 5.1 Hz, 1H).

### Step 2: Preparation of 7-bromo-3H-thieno[3,2-d]pyrimidin-4-one

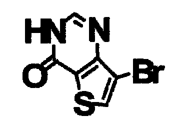

Thieno[3,2-*d*]pyrimidin-4(3*H*)-one (12.5 g) was dissolved in acetic acid (52 mL), and bromine (13 mL) was added thereto. The resulting mixture was stirred in a sealed container for 12 hrs at 120°C, cooled down to room temperature, and purified by distillation under reduced pressure to remove acetic acid. Ice water was added to the mixture, and a solid thus obtained was washed with ether and dried to obtain the title compound (7.8 g).
¹H NMR (300 MHz, DMSO-*d*₆): *δ* 12.75 (brs, 1H), 8.36 (s, 1H), 8.24 (s, 1H).

### Step 3: Preparation of 7-bromo-4-chlorothieno[3,2-d]pyrimidine

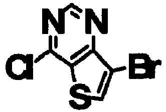

7-bromothieno[3,2-*d*]pyrimidin-4(3*H*)-one (5.9 g) was dissolved in POCl₃ (20 mL), and stirred for 3 hrs at 150°C. The mixture was cooled down to room temperature, and the remaining POCl₃ was concentrated and put into ice water. A solid thus obtained was washed with sodium bicarbonate solution, and then dried with nitrogen gas to yield the title compound. The solid was dried over anhydrous sodium sulfate, filtered and distilled under reduced pressure to obtain the title compound (1.0 g, Yield: 39%).
¹H NMR (300 MHz, DMSO-*d*₆): *δ* 9.16 (s, 1H), 8.79 (s, 1H).

The compound of Preparation Example 2 was prepared by using Reaction Scheme 2 below:

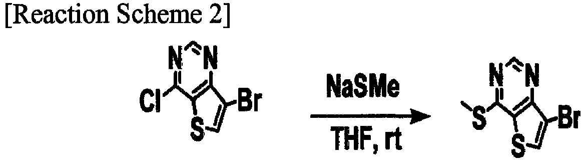

### Preparation Example 2: Preparation of 7-bromo-4-methylsulfonylthieno[3,2-d]pyrimidine

7-bromo-4-chlorothieno[3,2-*d*]pyrimidine (1 g, 4.0 mmol) prepared in Preparation Example 1 was put into tetrahydrofuran (30 mL), and then NaSMe (38 mg, 4.8 mmol) was added thereto at room temperature while stirring the mixture. After 12 hrs, the resulting solution was cooled down to less than 10°C, and then stirred for 1 hr. A solid thus formed was filtered and washed with water. The white solid compound thus obtained was dried with warm wind for 12 hrs at 45°C to yield the title compound (800 mg).
¹H NMR (300 MHz, DMSO-*d*₆): *δ* 9.08 (s, 1H), 8.57 (s, 1H), 2.77 (s, 3H).

The compound of Preparation Example 3 was prepared by using Reaction Scheme 3 below:

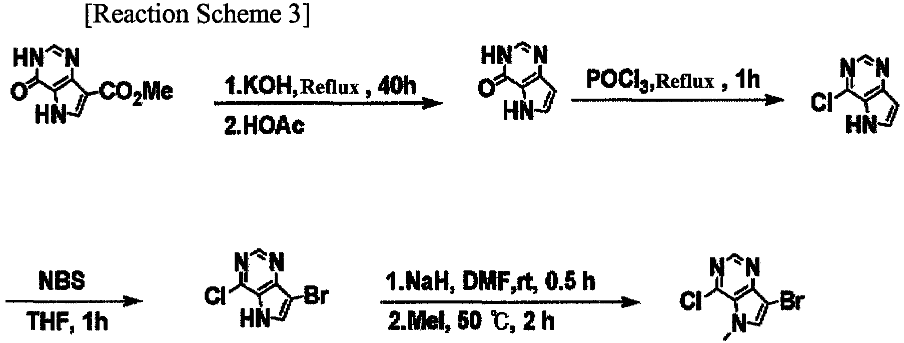

### Preparation Example 3: Preparation of 7-bromo-4-chloro-5-methyl-5H-pyrrolo[3,2-d]pyrimidine

### Step 1: Preparation of 3,5-dihydropyrrolo[3,2-d]pyrimidin-4-one

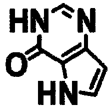

Methyl 4-oxo-4,5-dihydro-3*H*-pyrrolo[3,2-*d*]pyrimidin-7-carboxylate (20 g) was synthesized by using a conventional method (Organic Process Research & Development 2009, 13, 928-932). 10% potassium hydroxide (240 mL) was added thereto, and the mixture thus formed was stirred under reflux for 40 hrs. Upon completion of the reaction, the mixture was cooled down to room temperature, and neutralized to pH 6.5 to 7.5 by adding acetic acid. A solid thus formed was filtered and dried to obtain the title compound.
¹H NMR (300 MHz, DMSO-*d*₆): *δ* 12.05 (bs, 1 H), 11.82 (bs, 1 H), 7.77 (s, 1 H), 7.36 (s, 1 H), 6.35 (s, 1 H).

### Step 2: Preparation of 4-chloro-5H-pyrrolo[3,2-d]pyrimidine

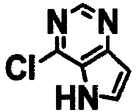

POCl₃ (5 mL) was added to 3,5-dihydropyrrolo[3,2-*d*]pyrimidin-4-one (1.8 g) and the mixture thus formed was stirred under reflux. Upon completion of the reaction, the mixture was distilled under reduced pressure to remove POCl₃. The resulting mixture, to which ethyl acetate was added, was washed with sodium carbonate, and then washed with brine. The mixture was dried over anhydrous sodium sulfate, filtered and distilled under reduced pressure to obtain the title compound.
¹H NMR (300 MHz, DMSO-*d*₆): *δ* 12.43 (s, 1 H), 8.61 (s, 1 H), 7.97 (dd, 1 H), 6.72 (dd, 1 H).

### Step 3: Preparation of 7-bromo-4-chloro-5H-pyrrolo[3,2-d]pyrimidine

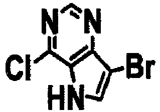

Tetrahydrofuran (5 mL) and N-bromosuccinimide (116 mg) were added to 4-chloro-5*H*-pyrrolo[3,2-*d*]pyrimidine (100 mg). The mixture was stirred for 1 hr and, upon completion of the reaction, ethyl acetate was added thereto, and washed with water. The mixture was dried over anhydrous sodium sulfate, filtered and distilled under reduced pressure to obtain the title compound.
¹H NMR (300 MHz, DMSO-*d*₆): *δ* 12.95 (s, 1 H), 8.71 (s, 1 H), 8.24 (d, 1 H)

### Step 4: Preparation of 7-bromo-4-chloro-5-methyl-5H-pyrrolo[3,2-d]pyrimidine

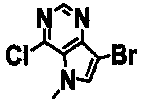

*N,N*-dimethylformamide (10 mL) and NaH (36 mg) were added to 7-bromo-4-chloro-5*H*-pyrrolo[3,2-*d*]pyrimidine (147 mg), followed by stirring for 30 mins. Subsequently, methyl iodide was added thereto, and the mixture was heated under stirring at 50°C for 2 hrs. Upon completion of the reaction, ethyl acetate was added thereto, and the mixture was washed with water. The resulting compound was dried over anhydrous sodium sulfate, filtered and distilled under reduced pressure to obtain the title compound.
¹H NMR (300 MHz, DMSO-*d*₆): *δ* 8.68(s, 1H), 8.22(s, 1H), 4.11 (s, 3H).

The compound of Example 1 was prepared according to the reaction scheme below:

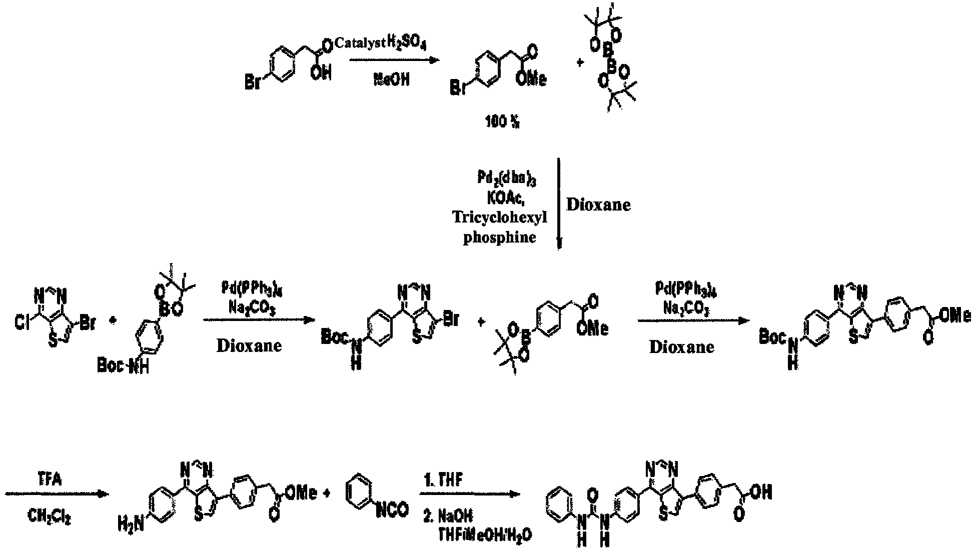

### Example 1: Preparation of 2-(4-(4-(4-(3-phenylureido)phenyl)thieno[3,2-d]pyrimidin-7-yl)phenyl)acetic acid

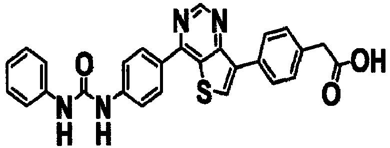

### Step 1: Preparation of methyl 2-(4-bromophenyl)acetate

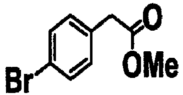

2-(4-bromophenyl)acetic acid (50 g) was introduced to methanol (50 mL), sulfuric acid (20 mL) was added thereto, followed by refluxing the mixture for 2 hrs. The reactant thus obtained was cooled down, and the solvent was concentrated to obtain the title compound (49 g).
¹H NMR (300 MHz, CDCl₃): 7.45 (d, 2H), 7.16 (d, 2H), 3.70 (s, 3H), 3.59 (s, 2H)

### Step 2: Preparation of methyl 2-(4-(4,4,5,5-tetramethyl-1,3,2-dioxaborolan-2-yl)phenyl)acetate

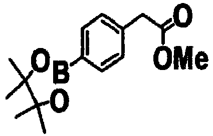

Methyl 2-(4-bromophenyl)acetate (43 g), Pd₂(dba)₃ (8.6 g), calcium acetate (27.6 g), tricyclohexylphosphine (6.3 g), bis(pinacolato)diborane (53 g) were placed in a reactor having 1,4-dioxane (430 mL), and the mixture was stirred under argon gas atmosphere. The reactor was heated to 90°C, followed by stirring for 12 hrs. The reactor was cooled, and the reactant thus obtained was extracted using acetate (500 mL) and water (500 mL). The reactant was dried over anhydrous magnesium sulfate, concentrated, and purified by column chromatography (dichloromethane : hexane = 3 : 1) to obtain the title compound (43 g).
¹H NMR (300 MHz, CDCl₃): 7.77(2H, d), 7.28 (2H, d), 3.68 (3H, s), 3.64 (2H, s), 1.34 (12H, s)

### Step 3: Reparation of tert-butyl 4-(7-(bromothieno[3,2-d]pyrimidin-4-yl)phenylcarbamate

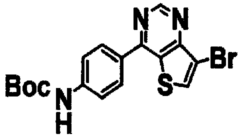

7-bromo-4-chlorothieno[3,2-*d*]pyrimidine (13 g) prepared in Preparation Example 1, *tert*-butyl 4-(4,4,5,5-tetramethyl-1,3,2-dioxaboran-2-yl)phenylcarbamate (20 g), tetrakis triphenylphosphine palladium (3.16 g) and sodium carbonate (8.28 g) were added in a mixed solution (100 mL) of 1,4-dioxane and water (4:1), and the mixture was placed under argon gas atmosphere, while stirring for 18 hrs at 80°C. Upon completion of the reaction, ethyl acetate (300 mL) and water (300 mL) were added thereto and the resulting mixture was extracted, dried over anhydrous magnesium sulfate, and then the solvent was concentrated. Methanol (30 mL) was added to the reactant, stirred for recrystallization, and then filtered to obtain the title compound in yellow (12.5 g).
¹H NMR (300 MHz, DMSO-*d*₆): *δ* 9.80 (s, 1H), 9.31 (s, 1H), 8.7 (s, 1H), 8.12 (d, 2H), 7.73 (d, 2H), 1.50 (s, 9H)

### Step 4: Preparation of methyl 2-(4-(4-(4-(tert-butoxycarbonylamino)phenyl)thieno[3,2-d]pyrimidin-7-yl)phenyl)acetate

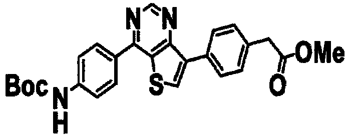

*tert*-Butyl 4-(7-bromothieno[3,2-*d*]pyrimidin-4yl)phenylcarbamate (6.18 g) prepared in Step 3, methyl 2-(4-(4,4,5,5-tetramethyl-1,3,2-dioxaborolan-2-yl)phenyl)acetate (5.3 g) prepared in Step 2, tetrakis triphenylphosphine palladium (1.05 g) and sodium carbonate (4.83 g) were added in a mixed solution (100 mL) of 1,4-dioxane and water (4:1), and the mixture was placed under argon gas atmosphere, while stirring for 18 hrs at 90°C. Upon completion of the reaction, ethyl acetate (300 mL) and water (300 mL) were added thereto and the resulting mixture was extracted, dried over anhydrous magnesium sulfate, and then the solvent was concentrated. Methanol was added to the reactant, which was stirred for recrystallization, and then filtered to obtain the title compound in yellow (4.47 g).
¹H NMR (300 MHz, DMSO-*d*₆): *δ* 9.79(s, 1H), 9.28(s, 1H), 8.72 (s, 1H), 8.14(d, 2H), 7.99(d, 2H), 7.73 (d, 2H), 7.36 (d, 2H), 3.71 (s, 2H), 3.60 (s, 3H), 1.50 (s, 9H)

### Step 5: Preparation of 2-(4-(4-(4-(3-phenylureido)phenyl)thieno[3,2-d]pyrimidin-7-yl)acetic acid

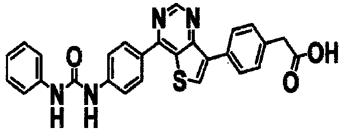

Methyl 2-(4-(4-(4-(*tert*-butoxycarbonylamino)phenyl)thieno[3,2-*d*]pyrimidin-7-yl)phenyl)acetate (1.12 g) prepared in Step 4 was introduced to dichloromethane (10 mL), to which trifluoroacetic acid (0.08 mL) was added, and the mixture was stirred for 2 hrs at room temperature. The mixture thus obtained was distilled under reduced pressure to remove the solvent, to which dichloromethane and an aqueous solution of sodium bicarbonate were added, and the mixture was stirred sufficiently. The organic layer thus formed was extracted. The organic layer was dried over anhydrous magnesium sulfate, and concentrated. The residue thus formed was dissolved in tetrahydrofuran, to which phenylisocyanate (0.36 g) was added, followed by stirring for 24 hrs at room temperature. The reaction solvent was concentrated, and diethyl ether was added for recrystallization. The resulting compound was filtered and the filtrate was added to a mixed solution of tetrahydrofuran, methanol and water (tetrahydrofuran:methanol:water = 1:1:1), stirred, and further stirred with the addition of sodium hydroxide (36 mg). The resulting mixture was distilled under reduced pressure to remove the solvent, and 1N HCl was added to adjust the pH in the range of 3 to 4. A solid was filtered and washed with water to obtain the title compound (1.1 g).
¹H NMR (300 MHz, DMSO-*d*₆): *δ* 12.39 (bs, 1H), 9.30 (s, 1H), 9.17 (s, 1H), 8.89 (s, 1H), 8.76 (s, 1H), 8.20 (d, 2H), 8.04 (d, 2H), 7.75 (d, 2H), 7.50 (d, 2H), 7.41 (d, 2H), 7.30 (t, 2H), 7.00 (t, 1H), 3.64 (s, 2H)

### Example 2: Preparation of 2-(4-(4-(4-(3-(3-chlorophenyl)ureido)phenyl)thieno[3,2-d]pyrimidin-7-yl) phenyl)acetic acid

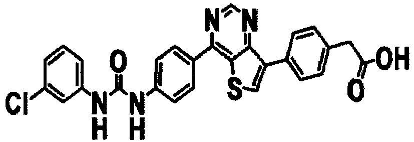

The procedure of Example 1 was repeated except that 3-chlorophenylisocyanate was used, instead of phenylisocyanate, in Step 5 to obtain the title compound.
¹H NMR (300 MHz, DMSO-*d*₆): *δ* 12.40 (bs, 1H), 9.31 (s, 1H), 9.23 (s, 1H), 9.08 (s, 1H), 8.76 (s, 1H), 8.20 (d, 2H), 8.04 (d, 2H), 7.76 (m, 3H), 7.41 (d, 2H), 7.32 (m, 2H), 7.05 (m, 1H), 3.63 (s, 2H).

### Example 3: Preparation of 4-(4-(4-(3-(3-chlorophenyl)ureido)phenyl)thieno[3,2-d]pyrimidin-7-yl)benzoic acid

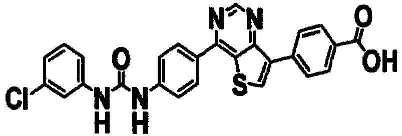

The procedure of Example 2 was repeated except that 4-bromobenzoic acid was used, instead of 2-(4-bromophenyl)acetic acid, in Step 1 to obtain the title compound.
¹H NMR (300 MHz, DMSO-*d*₆): *δ* 12.30 (bs, 1H), 9.34 (s, 1H), 9.24 (s, 1H), 9.08 (s, 1H), 8.95 (s, 1H), 8.27 (d, 2H), 8.20 (d, 2H), 8.07 (d, 2H), 7.75 (m, 3H), 7.32 (m, 2H), 7.06 (m, 1H)

### Example 4: Preparation of 2-(4-(4-(4-(3-(3,4-difluorophenyl)ureido)phenyl)thieno[3,2-d]pyrimidin-7-yl)phenyl)acetic acid

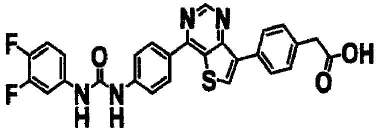

The procedure of Example 1 was repeated except that 3,4-difluorothiophenylisocyanate was used, instead of phenylisocyanate, in Step 5 to obtain the title compound.
¹H NMR (300 MHz, DMSO-*d*₆): *δ* 12.20 (bs, 1H), 9.29 (s, 1H), 9.21 (s, 1H), 9.07 (s, 1H), 8.74 (s, 1H), 8.19 (d, 2H), 8.03 (d, 2H), 7.73 (d, 2H), 7.69 (m, 1H), 7.40 (d, 2H), 7.34 (m, 1H), 7.13 (m, 1H), 3.63 (s, 2H)

### Example 5: Preparation of 2-(4-(4-(4-(3-(2-(trifluoromethoxy)phenyl)ureido)phenyl)thieno[3,2-d]pyrimidin-7-yl)phenyl)acetic acid

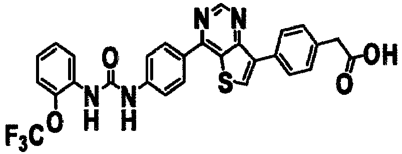

The procedure of Example 1 was repeated except that 2-trifluoromethoxyphenylisocyanate was used, instead of phenylisocyanate, in Step 5 to obtain the title compound.
¹H NMR (300 MHz, DMSO-*d*₆): *δ* 12.21 (bs, 1H), 9.68 (s, 1H), 9.30 (s, 1H), 8.75 (s, 1H), 8.63 (s, 1H), 8.27 (d, 1H), 8.21 (d, 2H), 8.03 (d, 2H), 7.76 (d, 2H), 7.40 (m, 4H), 7.11 (t, 1H), 3.63 (s, 2H)

### Example 6: Preparation of 2-(4-(4-(4-(3-(4-methoxyphenyl)ureido)phenyl)thieno[3,2-d]pyrimidin-7-yl)phenyl)acetic acid

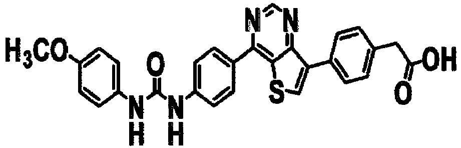

The procedure of Example 1 was repeated except that 4-methoxyphenylisocyanate was used, instead of phenylisocyanate, in Step 5 to obtain the title compound.
¹H NMR (300 MHz, DMSO-*d*₆): *δ* 12.35 (bs, 1H), 9.30 (s, 1H), 9.11 (s, 1H), 8.78 (s, 1H), 8.72 (s, 1H), 8.19 (d, 2H), 8.05 (d, 2H), 7.76 (d, 2H), 7.38 (m, 4H), 6.88 (d, 2H), 3.70 (s, 3H), 3.62 (s, 2H)

### Example 7: Preparation of 3-(4-(4-(4-(3-(3-chlorophenyl)ureido)phenyl)thieno[3,2-d]pyrimidin-7-yl)phenyl)propanoic acid

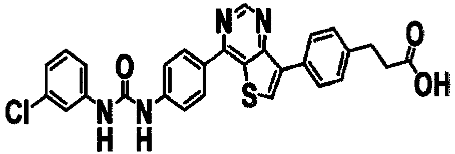

The procedure of Example 2 was repeated except for using 4-bromophenylpropanoic acid instead of 2-(4-bromopheyl)acetic acid in Step 1 to obtain the title compound.
¹H NMR (300 MHz, DMSO-*d*₆): *δ* 12.17 (bs, 1H), 9.32 (s, 1H), 9.30 (s, 1H), 9.19 (s, 1H), 8.73 (s, 1H), 8.20 (d, 2H), 8.01 (d, 2H), 7.76 (m, 3H), 7.38 (d, 2H), 7.32 (d, 2H), 7.04 (m, 1H), 2.90 (t, 2H), 2.59 (t, 2H)

### Example 8: Preparation of 2-(4-(4-(4-(3-ethylureido)phenyl)thieno[3,2-d]pyrimidin-7-yl)phenyl)acetic acid

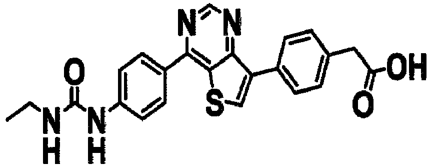

The procedure of Example 1 was repeated except for using ethylisocyanate instead of phenylisocyanate in Step 5 to obtain the title compound.
¹H NMR (300 MHz, DMSO-*d*₆): *δ* 12.35 (bs, 1H), 9.28 (s, 1H), 8.89 (s, 1H), 8.75 (s, 1H), 8.15 (d, 2H), 8.03 (d, 2H), 7.68 (d, 2H), 7.40 (d, 2H), 6.29 (t, 1H), 3.65 (s, 3H), 3.14 (m, 2H), 1.08 (t, 3H)

### Example 9: Preparation of methyl 2-(4-(4-(4-(3-(3-chlorophenyl)ureido)phenyl)thieno[3,2-d]pyrimidin-7-yl)phenyl)acetate

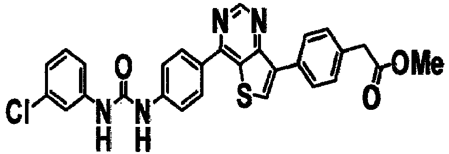

The procedure of Example 2 was repeated except for omitting the hydrolysis reaction in Step 5 to obtain the title compound.
¹H NMR (300 MHz, DMSO-d₆): *δ* 9.41 (s, 1H), 9.32 (s, 1H), 9.28 (s, 1H), 8.78 (s, 1H), 8.19 (d, 2H), 8.05 (d, 2H), 7.76 (m, 3H), 7.43 (d, 2H), 7.32 (m, 2H), 7.05 (m, 1H), 3.77 (s, 2H), 3.65 (s, 3H)

### Example 10: Preparation of 2-(4-(4-(4-(3-(2-chlorophenyl)ureido)phenyl)thieno[3,2-d]pyrimidin-7-yl)phenyl)acetic acid

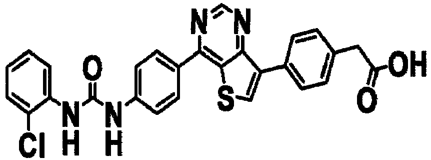

The procedure of Example 1 was repeated except for using 2-chlorophenylisocyanate instead of phenylisocyanate in Step 5 to obtain the title compound.
¹H NMR (300 MHz, DMSO-*d*₆): *δ* 12.30 (bs, 1H), 9.83 (s, 1H), 9.30 (s, 1H), 8.76 (s, 1H), 8.47 (s, 1H), 8.21 (d, 2H), 8.18 (d, 1H), 8.03 (d, 2H), 7.76 (d, 2H), 7.47 (d, 1H), 7.40 (d, 2H), 7.32 (t, 1H), 7.05 (t, 1H), 3.63 (s, 2H)

### Example 11: Preparation of 2-(4-(4-(4-(3-(4-chlorophenyl)ureido)phenyl)thieno[3,2-d]pyrimidin-7-yl)phenyl)acetic acid

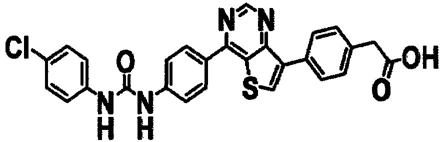

The procedure of Example 1 was repeated except for using 4-chlorophenylisocyanate instead of phenylisocyanate in Step 5 to obtain the title compound.
¹H NMR (300 MHz, DMSO-*d*₆): *δ* 12.45 (bs, 1H), 9.29 (s, 1H), 9.18 (s, 1H), 9.00 (s, 1H), 8.75 (s, 1H), 8.19 (d, 2H), 8.02 (d, 2H), 7.74 (d, 2H), 7.52 (d, 2H), 7.40 (d, 2H), 7.33 (d, 2H), 3.63 (s, 2H)

### Example 12: Preparation of 4-(4-(4-(4-(3-(3-chlorophenyl)ureido)phenyl)thieno[3,2-d]pyrimidin-7-yl)phenyl)butanoic acid

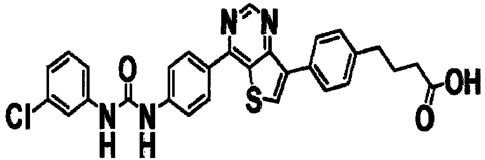

The procedure of Example 2 was repeated except for using 4-bromophenylbutanoic acid instead of 2-(4-bromophenyl)acetic acid in Step 1 to obtain the title compound.
¹H NMR (300 MHz, DMSO-*d*₆): *δ* 12.20 (br, 1H), 9.32(s, 1H), 9.25(s, 1H), 9.10(s, 1H), 8.73(s, 1H), 8.20(d, 2H), 8.03(d, 2H), 7.77)dd, 3H), 7.34(t, 4H), 7.06(m, 1H), 2.66(t, 2H), 2.27(t, 2H), 1.87(m, 2H)

### Example 13: Preparation of 3-(4-(4-(4-(3-(2-chlorophenyl)ureido)phenyl)thieno[3,2-d]pyrimidin-7-yl)phenyl)propanoic acid

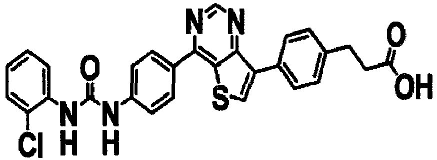

The procedure of Example 7 was repeated except for using 2-chlorophenylisocyanate instead of 2-chlorophenylisocyanate in Step 5 to obtain the title compound.
¹H NMR (300 MHz, DMSO-*d*₆): *δ* 12.20 (bs, 1H), 9.89 (s, 1H), 9.31 (s, 1H), 8.74 (s, 1H), 8.54 (s, 1H), 8.20 (m, 3H), 7.80 (d, 2H), 7.77 (d, 2H), 7.49 (d, 1H), 7.38 (d, 2H), 7.33 (d, 1H), 7.07 (t, 1H), 2.89 (t, 2H), 2.59 (t, 2H)

### Example 14: Preparation of 3-(4-(4-(4-(3-(3,4-difluorophenyl)ureido)phenyl)thieno[3,2-d]pyrimidin-7-yl)phenyl)propanoic acid

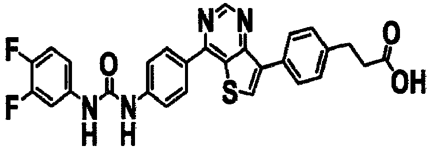

The procedure of Example 7 was repeated except for using 3,4-difluoroisocyanate instead of 2-chlorophenylisocyanate in Step 5 to obtain the title compound.
¹H NMR (300 MHz, DMSO-*d*₆): *δ* 12.18 (bs, 1H), 9.31 (s, 1H), 9.27 (s, 1H), 9.14 (s, 1H), 8.74 (s, 1H), 8.20 (d, 2H), 8.01 (d, 2H), 7.75 (d, 2H), 7.68 (m, 1H), 7.37 (m, 3H), 7.18 (m, 1H), 2.89 (t, 2H), 2.60 (t, 2H)

### Example 15: Preparation of 3-(4-(4-(4-(3-(4-methoxyphenyl)ureido)phenyl)thieno[3,2-d]pyrimidin-7-yl)phenyl)propanoic acid

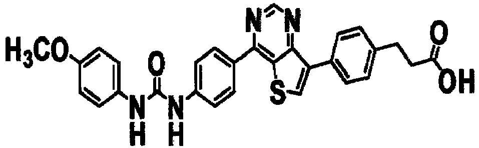

The procedure of Example 7 was repeated except for using 4-methoxyphenylisocyanate instead of 2-chlorophenylisocyanate in Step 5 to obtain the title compound.
¹H NMR (300 MHz, DMSO-*d*₆): *δ* 12.18 (bs, 1H), 9.30 (s, 1H), 9.12 (s, 1H), 8.73 (s, 2H), 8.19 (d, 2H), 8.00 (d, 2H), 7.75 (d, 2H), 7.39 (m, 4H), 6.90 (d, 2H), 3.72 (s, 3H), 2.90 (t, 2H), 2.59 (t, 2H)

### Example 16: Preparation of 2-(4-(4-(4-(3-cyclohexylureido)phenyl)thieno[3,2-d]pyrimidin-7-yl)phenyl)acetic acid

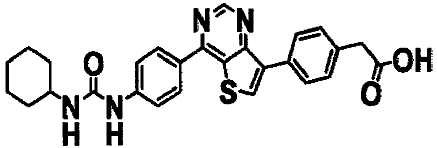

The procedure of Example 1 was repeated except for using cyclohexylisocyanate instead of phenylisocyanate in Step 5 to obtain the title compound.
¹H NMR (300 MHz, DMSO-*d*₆): *δ* 9.26(s, 1H), 8.92(s, 1H), 8.71(s, 1H), 8.12(d, 2H), 8.02(d, 2H), 7.64(d, 2H), 7.38(d, 2H), 6.42(d, 1H) 3.60(s, 2H), 1.83∼1.79(m, 2H), 1.68∼1.64(m, 2H), 1.56∼1.51(m, 1H), 1.33∼1.16(m, 6H)

### Example 17: Preparation of 3-(4-(4-(4-(3-(2-chloro-6-methylphenyl)ureido)phenyl)thieno[3,2-d]pyrimidin-7-yl)phenyl)propanoic acid

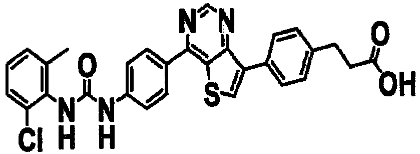

The procedure of Example 7 was repeated except for using 2-chloro-6-methylphenylisocyanate instead of phenylisocyanate in Step 5 to obtain the title compound.
¹H NMR (300 MHz, DMSO-*d*₆): *δ* 12.16(s, 1H), 9.35(s, 1H), 9.29(s, 1H), 8.72(s, 1H), 8.17(d, 2H), 8.00(d, 2H), 7.75(d, 2H), 7.36(m, 4H), 7.30∼7.12(m, 3H), 3.29(s, 3H), 2.87(t, 2H), 2.59(t, 2H)

### Example 18: Preparation of 3-(4-(4-(4-(3-(3-chloro-4-methoxyphenyl)ureido)phenyl)thieno[3,2-d]pyrimidin-7-yl)phenyl)propanoic acid

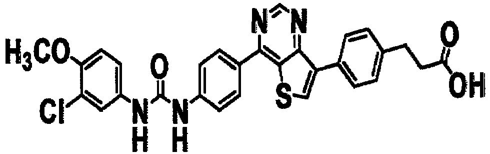

The procedure of Example 7 was repeated except for using 3-chloro-4-methoxyphenylisocyanate instead of phenylisocyanate in Step 5 to obtain the title compound.
¹H NMR (300 MHz, DMSO-*d*₆): *δ* 12.15(s, 1H), 9.31(s 1H), 9.19(s, 1H), 8.88(s, 1H), 8.73(s, 1H), 8.19(d, 2H), 8.01(d, 2H), 7.75(d, 2H), 7.61(s, 1H), 7.38(d, 2H), 7.20(d, 1H), 7.11(d, 1H), 3.82(s, 3H) 2.90(t, 2H), 2.59(t, 2H)

### Example 19: Preparation of 3-(4-(4-(4-(3-(3-methoxyphenyl)ureido)phenyl)thieno[3,2-d]pyrimidin-7-yl)phenyl)propanoic acid

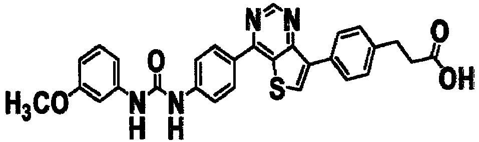

The procedure of Example 7 was repeated except for using 3-methoxyphenylisocyanate instead of phenylisocyanate in Step 5 to obtain the title compound.
¹H NMR (300 MHz, DMSO-*d*₆): *δ* 1.2.15 (bs, 1H), 9.28 (s, 1H), 9.18 (s, 1H), 8.93 (s, 1H), 8.71 (s, 1H), 8.19 (d, 2H), 8.00 (d, 2H), 7.75 (d, 2H), 7.38 (d, 2H), 7.19 (m, 2H), 6.97 (d, 1H), 6.57 (d, 1H), 3.74 (s, 3H), 2.89 (t, 2H), 2.58 (t, 2H)

### Example 20: Preparation of 3-(4-(4-(4-(3-(2-methoxyphenyl)ureido)phenyl)thieno[3,2-d]pyrimidin-7-yl)phenyl)propanoic acid

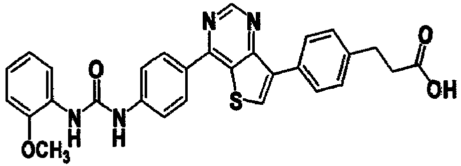

The procedure of Example 7 was repeated except for using 2-methoxyphenylisocyanate instead of phenylisocyanate in Step 5 to obtain the title compound.
¹H NMR (300 MHz, DMSO-*d*₆): *δ* 12.20 (bs, 1H), 9.74 (s, 1H), 9.29 (s, 1H), 8.73 (s, 1H), 8.38 (s, 1H), 8.20 (d, 2H), 8.15 (d, 1H), 8.00 (d, 2H), 7.75 (d, 2H), 7.38 (d, 2H), 6.95 (m, 3H), 3.89 (s, 3H), 2.88 (t, 2H), 2.58 (t, 2H)

### Example 21: Preparation of 3-(4-(4-(4-(3-p-tolylureido)phenyl)thieno[3,2-d]pyrimidin-7-yl)phenyl)propanoic acid

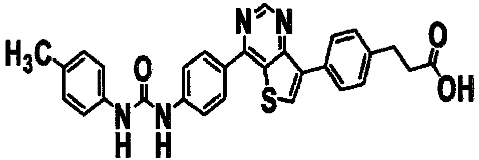

The procedure of Example 7 was repeated except for using 4-methylphenylisocyanate instead of phenylisocyanate in Step 5 to obtain the title compound.
¹H NMR (300 MHz, DMSO-*d*₆): *δ* 12.16 (bs, 1H), 9.29 (s, 1H), 9.19 (s, 1H), 8.85 (s, 1H), 8.72 (s, 1H), 8.18 (d, 2H), 8.00 (d, 2H), 7.74 (d, 2H), 7.38 (d, 4H), 7.10 (d, 2H), 2.89 (t, 2H), 2.59 (t, 2H), 2.25 (s, 3H)

### Example 22: Preparation of 3-(4-(4-(4-(3-(5-fluoro-2-methylphenyl)ureido)phenyl)thieno[3,2-d]pyrimidin-7-yl)phenyl)propanoic acid

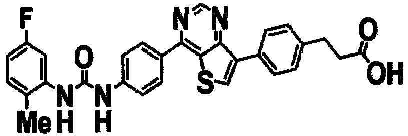

The procedure of Example 7 was repeated except for using 5-fluoro-2-methylphenylisocyanate instead of phenylisocyanate in Step 5 to obtain the title compound.
¹H NMR (300 MHz, DMSO-*d*₆): *δ* 9.86(s, 1H), 9.29(s, 1H), 8.73(s, 1H), 8.36(s, 1H), 8.20(d, 2H), 7.99(d, 2H), 7.86(d, 1H), 7.76(d, 1H), 7.37(d, 2H), 7.19(t, 1H), 6.76(t, 1H), 2.85(t, 2H), 2.59(t, 2H)

### Example 23: Preparation of 3-(4-(4-(4-(3-(2-fluoro-5-(trifluoromethyl)phenyl)ureido)phenyl)thieno[3,2-d]pyrimidin-7-yl)phenyl)propanoic acid

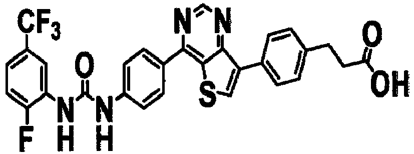

The procedure of Example 7 was repeated except for using 2-fluoro-5-(trifluoromethyl)phenylisocyanate instead of phenylisocyanate in Step 5 to obtain the title compound.
¹H NMR (300 MHz, DMSO-*d*₆): *δ* 12.17(s, 1H), 9.61(s, 1H), 9.31(s, 1H), 9.07(s, 1H), 8.34(s, 1H), 8.64(d, 1H), 8.23(d, 2H), 7.78(d, 2H), 7.53(t, 1H), 7.38(t, 3H), 2.90(t, 2H), 2.60(t, 2H)

### Example 24: Preparation of 3-(4-(4-(4-(3-(3,4,5-trimethoxyphenyl)ureido)phenyl)thieno[3,2-d]pyrimidin-7-yl)phenyl)propanoic acid

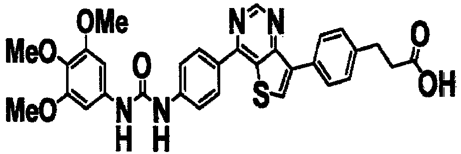

The procedure of Example 7 was repeated except for using 3,4,5-trimethoxyphenylisocyanate instead of phenylisocyanate in Step 5 to obtain the title compound.
¹H NMR (300 MHz, DMSO-*d*₆): *δ* 9.31(s, 1H), 9.08(s, 1H), 8.81(s, 1H), 8.74(s, 1H), 8.20(d, 2H), 8.01(d, 2H), 7.76(d, 2H), 3.78(s, 6H), 3.62(s, 3H), 2.90(t, 2H), 2.60(t, 2H)

### Example 25: Preparation of 3-(4-(4-(4-(3-(2,3-dichlorophenyl)ureido)phenyl)thieno[3,2-d]pyrimidin-7-yl)phenyl)propanoic acid

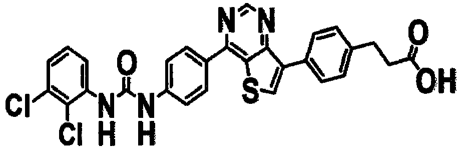

The procedure of Example 7 was repeated except for using 2,3-dichlorophenylisocyanate instead of phenylisocyanate in Step 5 to obtain the title compound.
¹H NMR (300 MHz, DMSO-*d*₆): *δ* 12.15 (bs, 1H), 9.89 (s, 1H), 9.30 (s, 1H), 8.73 (s, 1H), 8.63 (s, 1H), 8.20 (m, 3H), 8.00 (d, 2H), 7.75 (d, 2H), 7.36 (m, 4H), 2.88 (t, 2H), 2.58 (t, 2H)

### Example 26: Preparation of 3-(4-(4-(4-(3-(3-(trifluoromethyl)phenyl)ureido)phenyl)thieno[3,2-d]pyrimidin-7-yl)phenyl)propanoic acid

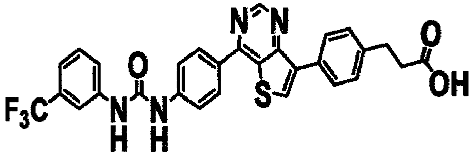

The procedure of Example 7 was repeated except for using 3-(trifluoromethyl)phenylisocyanate instead of phenylisocyanate in Step 5 to obtain the title compound.
¹H NMR (300 MHz, DMSO-*d*₆): *δ* 12.14 (bs, 1H), 9.29 (s, 1H), 9.26 (s, 1H), 9.23 (s, 1H), 8.72 (s, 1H), 8.20 (d, 2H), 8.04 (s, 1H), 8.00 (d, 2H), 7.76 (d, 2H), 7.60 (d, 1H), 7.53 (t, 1H), 7.36 (m, 3H), 2.88 (t, 2H), 2.58 (t, 2H)

### Example 27: Preparation of 3-(4-(4-(4-(3-cyclohexylureido)phenyl)thieno[3,2-d]pyrimidin-7-yl)phenyl)propanoic acid

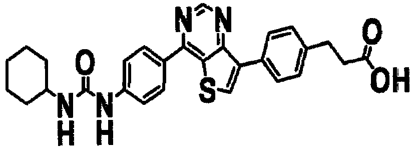

The procedure of Example 7 was repeated except for using cyclohexylisocyanate instead of phenylisocyanate in Step 5 to obtain the title compound.
¹H NMR (300 MHz, DMSO-*d*₆): *δ* 9.26(s, 1H), 8.92(s, 1H), 8.71(s, 1H), 8.12(d, 2H), 8.02(d, 2H), 7.64(d, 2H), 7.38(d, 2H), 6.42(d, 1H), 2.90(t, 2H), 2.60(t, 2H) 1.83~1.79(m, 2H), 1.68~1.64(m, 2H), 1.56∼1.51(m, 1H), 1.33∼1.16(m, 6H)

### Example 28: Preparation of 3-(4-(4-(4-(3-(3-chlorophenyl)ureido)phenyl)thieno[3,2-d]pyrimidin-7-yl)phenyl)propanamide

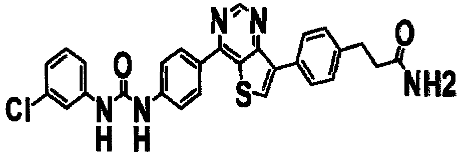

3-(4-(4-(4-(3-(3-chlorophenyl)ureido)phenyl)thieno[3,2-*d*]pyrimidin-7-yl)phenyl)propanoic acid (100 mg) prepared in Example 7, 0.5 M dioxane/ammonia solution (2 mL), HATU (76 mg) and *N,N-*diisopropylethylamine (0.035 mL) were added to dimethylformamide (5 mL), and the mixture was stirred overnight at room temperature. Upon completion of the reaction, ethylacetate and water were added thereto, and the organic layer thus formed was separated. The organic layer was dried over anhydrous sodium sulfate, filtered and distilled under reduced pressure, and purified by column chromatography to obtain the title compound (65 mg).
¹H NMR (300 MHz, DMSO-*d*₆): *δ* 9.32 (s, 1H), 9.22 (s, 1H), 9.07 (s, 1H), 8.75 (s, 1H), 8.21 (d, 2H), 8.02 (d, 2H), 7.78 (m, 3H), 7.34 (m, 4H), 7.05 (m, 1H), 6.80 (s, 1H), 2.88 (t, 2H), 2.42 (t, 2H)

### Example 29: Preparation of 1-(3-chlorophenyl)-3-(4-(7-(4-(3-hydroxypropyl)phenyl)thieno[3,2-d]pyrimidin-4-yl)phenyl)urea

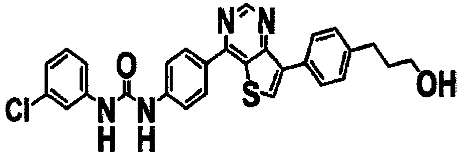

3-(4-(4-(4-(3-(3-chlorophenyl)ureido)phenyl)thieno[3,2-*d*]pyrimidin-7-yl)phenyl)propanoic acid (100 mg) prepared in Example 7 was added to dichloromethane (5 mL), and the mixture was cooled down to -78°C, and DIBAL-H (0.05 mL) was slowly added thereto. The temperature of the mixture was raised to 0°C, and the mixture was stirred overnight, followed by washing with water. The compound thus obtained was dried over anhydrous sodium sulfate, filtered and distilled under reduced pressure, and purified by column chromatography to obtain the title compound (53 mg).
¹H NMR (300 MHz, DMSO-*d*₆): *δ* 9.31 (s, 1H), 9.23 (s, 1H), 9.08 (s, 1H), 8.73 (s, 1H), 8.20 (d, 2H), 8.02 (d, 2H), 7.77 (m, 3H), 7.34 (m, 4H), 7.04 (m, 1H), 4.50 (t, 1H), 3.45 (q, 2H), 2.68 (t, 2H), 1.77 (t, 2H)

### Example 30: Preparation of 3-(4-(4-(4-(3-(3-chlorophenyl)ureido)phenyl)thieno[3,2-d]pyrimidin-7-yl)-2-fluorophenyl)propanoic acid

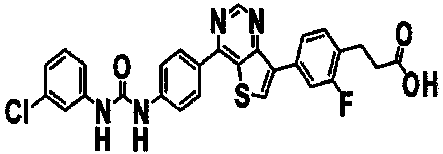

The procedure of Example 7 was repeated except for using 3-(4-bromo-2-fluorophenyl)propanoic acid instead of 4-bromophenylpropanoic acid in Step 1 to obtain the title compound.
¹H NMR (300 MHz, DMSO-*d*₆): *δ* 12.22 (bs, 1H), 9.33 (s, 1H), 9.24 (s, 1H), 9.08 (s, 1H), 8.87 (s, 1H), 8.20 (d, 2H), 8.02 (d, 1H), 7.90 (d, 1H), 7.62 (m, 3H), 7.46 (t, 1H), 7.32 (m, 2H), 7.05 (m, 1H), 2.92 (t, 2H), 2.60 (t, 2H)

### Example 31: Preparation of 1-(3-chlorophenyl)-3-(4-(7-(4-(methylsulfon)phenyl)thieno[3,2-d]pyrimidin-4-yl)phenyl)urea

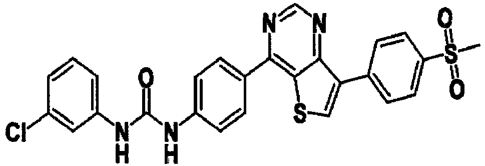

The procedure of Example 7 was repeated except for using 1-bromo-4-(methylsulfonyl)benzene instead of 4-bromophenylpropanoic acid in Step 1 to obtain the title compound.
¹H NMR (300 MHz, DMSO-*d*₆): *δ* 9.36(s, 1H), 9.25(s, 1H), 9.21(s, 1H), 9.03(s, 1H), 8.40(d, 2H), 8.22(d, 2H), 8.09(d, 2H), 7.77(t, 2H), 7.33(d, 2H), 6.79(s, 1H), 3.29(s, 3H)

The compound of Example 32 was prepared by using the reaction scheme below:

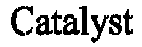

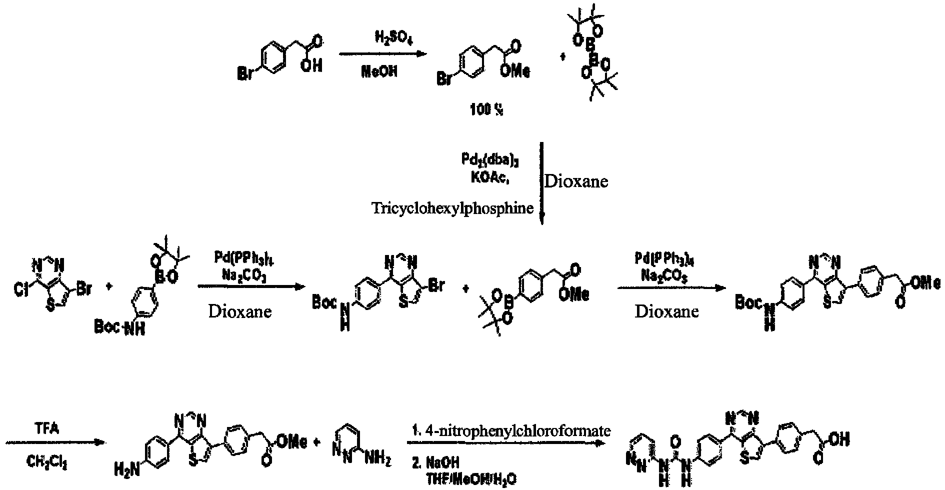

### Example 32: Preparation of 2-(4-(4-(4-(3-pyridazin-3-ylureido)phenyl)thieno[3,2-d]pyrimidin-7-yl)phenyl)acetic acid

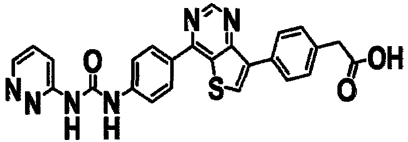

Methyl 2-(4-(4-(4-(*tert*-butoxycarbonylamino)phenyl)thieno[3,2-*d*]pyrimidin-7-yl)phenyl)acetate (50 mg) prepared in Step 4 of Example 1, 4-nitrophenylchloroformate (28.1 mg) and 3-aminopyridazine (17 mg) were added to 1,4-dioxane (3 mL), and the mixture was stirred overnight at room temperature. The reaction solvent was concentrated, and diethyl ether was added for recrystallization. The resulting compound was filtered and the filtrate was added to a mixed solution of tetrahydrofuran, methanol and water (tetrahydrofuran:methanol:water = 1:1:1), stirred, and further stirred with the addition of sodium hydroxide (16 mg). The resulting mixture was distilled under reduced pressure to remove the solvent, and 1N HCl was added to adjust the pH in the range of 3 to 4. A solid thus obtained was filtered and washed with water to obtain the title compound (40 mg).
¹H NMR (300 MHz, DMSO-*d*₆): *δ* 12.38(s, 1H), 10.11(s. 1H), 9.89(s, 1H), 9.33(s, 1H), 8.91(d, 1H), 8.78(d, 1H), 8.24(d, 2H), 8.07(t, 3H), 7.81(d, 2H), 7.68(q, 1H), 7.42(d, 2H), 3.65(s, 2H)

### Example 33: Preparation of 2-(4-(4-(4-(3-pyridin-3-ylureido)phenyl)thieno[3,2-d]pyrimidin-7-yl)phenyl)acetic acid

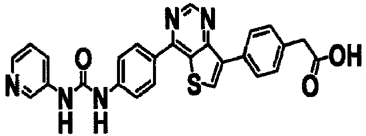

The procedure of Example 32 was repeated except for using 3-aminopyridine instead of 3-aminopyridazine to obtain the title compound.
¹H NMR (300 MHz, DMSO-*d*₆): *δ* 12.38 (bs, 1H), 9.31 (m, 2H), 9.06 (s, 1H), 8.75 (s, 1H), 8.64 (s, 1H), 8.20 (m, 3H), 8.03 (d, 2H), 7.97 (d, 1H), 7.76 (d, 2H), 7.40 (d, 2H), 7.34 (m, 1H), 3.64 (s, 2H)

### Example 34: Preparation of 2-(4-(4-(4-(3-(2-chloropyridin-4-yl)ureido)phenyl)thieno[3,2-d]pyrimidin-7-yl)phenyl)acetic acid

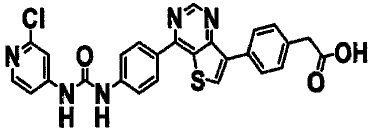

The procedure of Example 32 was repeated except for using 3-amino-2-chloropyridine instead of 3-aminopyridazine to obtain the title compound.
¹H NMR (300 MHz, DMSO-*d*₆): *δ* 12.38 (bs, 1H), 9.70 (s, 1H), 9.59 (s, 1H), 9.29 (s, 1H), 8.74 (s, 1H), 8.19 (m, 3H), 8.03 (d, 2H), 7.75 (d, 2H), 7.67 (s, 1H), 7.38 (m, 3H), 3.57 (s, 2H)

### Example 35: Preparation of 2-(4-(4-(4-(3-(4-(dimethylamino)phenyl)ureido)phenyl)thieno[3,2-d]pyrimidin-7-yl)phenyl)acetic acid

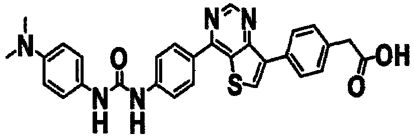

The procedure of Example 32 was repeated except for using 4-(dimethylamino)aniline instead of 3-aminopyridazine to obtain the title compound.
¹H NMR (300 MHz, DMSO-*d*₆): *δ* 12.36 (bs, 1H), 9.28 (s, 1H), 8.96 (s, 1H), 8.74 (s, 1H), 8.46 (s, 1H), 8.17 (d, 2H), 8.03 (d, 2H), 7.72 (d, 2H), 7.39 (d, 2H), 7.27 (d, 2H), 6.70 (m, 2H), 3.63 (s, 2H), 2.83 (s, 6H)

### Example 36: Preparation of 2-(4-(4-(4-(3-(4-chloropyridin-2-yl)ureido)phenyl)thieno[3,2-d]pyrimidin-7-yl)phenyl)acetic acid

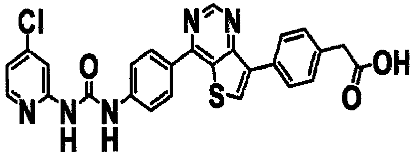

The procedure of Example 32 was repeated except for using 2-amino-4-chloropyridine instead of 3-aminopyridazine to obtain the title compound.
¹H NMR (300 MHz, DMSO-*d*₆): *δ* 12.40 (bs, 1H), 10.32 (s, 1H), 9.71 (s, 1H), 9.31 (s, 1H), 8.77 (s, 1H), 8.30 (d, 1H), 8.23 (d, 2H), 8.05 (d, 2H), 7.80 (m, 3H), 7.42 (d, 2H), 7.18 (d, 1H), 3.64 (s, 2H)

### Example 37: Preparation of 2-(4-(4-(4-(3-pyridin-2-ylureido)phenyl)thieno[3,2-d]pyrimidin-7-yl)phenyl)acetic acid

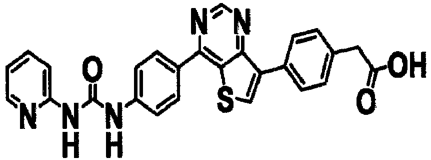

The procedure of Example 32 was repeated except for using 2-aminopyridine instead of 3-aminopyridazine to obtain the title compound.
¹H NMR (300 MHz, DMSO-*d*₆): *δ* 10.86 (s, 1H), 9.60 (s, 1H), 9.32 (s, 1H), 9.30 (s, 1H), 8.77 (s, 1H), 8.32 (d, 1H), 8.23 (d, 2H), 8.05 (d, 2H), 7.83 (m, 3H), 7.55 (d, 1H), 7.42 (d, 2H), 7.07 (t, 1H), 3.65 (s, 2H)

### Example 38: Preparation of 2-(4-(4-(4-(3-pyridin-4-ylureido)phenyl)thieno[3,2-d]pyrimidin-7-yl)phenyl)acetic acid

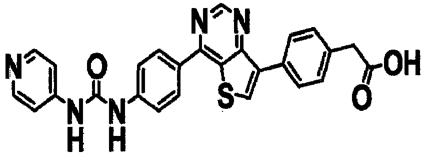

The procedure of Example 32 was repeated except for using 4-aminopyridine instead of 3-aminopyridazine to obtain the title compound.
¹H NMR (300 MHz, DMSO-*d*₆): *δ* 12.35 (bs, 1H), 9.76 (s, 1H), 9.70 (s, 1H), 9.30 (s, 1H), 8.73 (s, 1H), 8.37 (d, 1H), 8.19 (m, 3H), 8.04 (d, 2H), 7.76 (m, 3H), 7.49 (d, 1H), 7.41 (d, 2H), 3.63 (s, 2H)

### Example 39: Preparation of 3-(4-(4-(4-(3-(3-fluorophenyl)ureido)phenyl)thieno[3,2-d]pyrimidin-7-yl)phenyl)propanoic acid

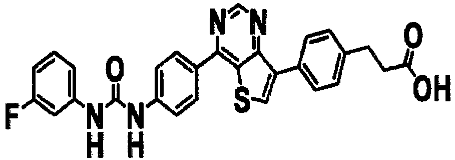

The procedure of Example 1 was repeated except for using 4-bromophenyl propanoic acid instead of 2-(4-bromophenyl)acetic acid in Step 1 and using 3-fluoroaniline instead of 3-aminopyridazine in Step 5 to obtain the title compound.
¹H NMR (300 MHz, DMSO-*d*₆): *δ* 12.15 (bs, 1H), 9.38 (s, 2H), 9.28 (s, 1H), 8.71 (s, 1H), 8.19 (d, 2H), 7.99 (d, 2H), 7.75 (d, 2H), 7.52 (d, 1H), 7.38 (d, 2H), 7.30 (m, 1H), 7.16 (d, 1H), 6.80 (t, 1H), 2.89 (t, 2H), 2.58 (t, 2H)

### Example 40: Preparation of 3-(4-(4-(4-(3-(4-(dimethylamino)phenyl)ureido)phenyl)thieno[3,2-d]pyrimidin-7-yl)phenyl)propanoic acid

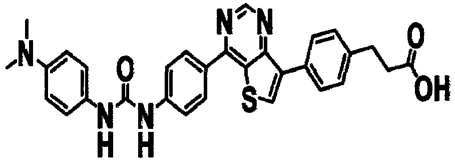

The procedure of Example 39 was repeated except for using 4-(dimethylamino)aniline instead of 3-fluoroaniline to obtain the title compound.
¹H NMR (300 MHz, DMSO-*d*₆): *δ* 12.16 (bs, 1H), 9.29 (s, 1H), 9.03 (s, 1H), 8.73 (s, 1H), 8.51 (s, 1H), 8.18 (d, 2H), 8.00 (d, 2H), 7.73 (d, 2H), 7.38 (d, 2H), 7.29 (d, 2H), 6.71 (d, 2H), 2.84 (m, 8H), 2.59 (t, 2H)

### Example 41: Preparation of 3-(4-(4-(4-(3-(2,3-difluorophenyl)ureido)phenyl)thieno[3,2-d]pyrimidin-7-yl)phenyl)propanoic acid

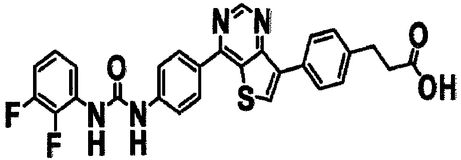

The procedure of Example 39 was repeated except for using 2,3-difluoroaniline instead of 3-fluoroaniline to obtain the title compound.
¹H NMR (300 MHz, DMSO-*d*₆): *δ* 12.18 (bs, 1H), 9.66 (s, 1H), 9.20 (s, 1H), 8.97 (s, 1H), 8.73 (s, 1H), 8.20 (d, 2H), 7.99 (m, 3H), 7.76 (d, 2H), 7.38 (d, 2H), 7.11 (m, 2H), 2.94 (t, 2H), 2.59 (t, 2H)

### Example 42: Preparation of 3-(4-(4-(4-(3-(4-chloropyridin-2-yl)ureido)phenyl)thieno[3,2-d]pyrimidin-7-yl)phenyl)propanoic acid

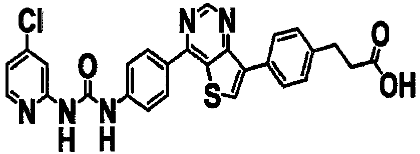

The procedure of Example 39 was repeated except for using 2-amino-4-chloropyridine instead of 3-fluoroaniline to obtain the title compound.
¹H NMR (300 MHz, DMSO-*d*₆): *δ* 12.17 (bs, 1H), 10.34 (s, 1H), 9.71 (s, 1H), 9.31 (s, 1H), 8.74 (s, 1H), 8.30 (d, 1H), 8.23 (d, 2H), 8.01 (d, 2H), 7.81 (m, 3H), 7.38 (d, 2H), 7.19 (m, 1H), 2.89 (t, 2H), 2.60 (t, 2H)

### Example 43: Preparation of 3-(4-(4-(4-(3-(2,5-difluorophenyl)ureido)phenyl)thieno [3,2-d]pyrimidin-7-yl)phenyl)propanoic acid

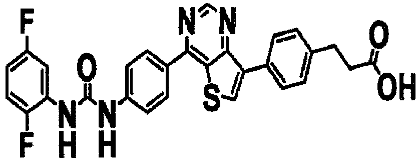

The procedure of Example 39 was repeated except for using 2,5-difluoroaniline instead of 3-fluoroaniline to obtain the title compound.
¹H NMR (300 MHz, DMSO-*d*₆): *δ* 12.21 (bs, 1H), 9.60 (s, 1H), 9.31 (s, 1H), 8.96 (s, 1H), 8.74 (s, 1H), 8.23 (d, 2H), 8.06 (m, 1H), 8.01 (d, 2H), 7.76 (d, 2H), 7.38 (d, 2H), 7.30 (m, 1H), 6.86 (m, 1H), 2.89 (t, 2H), 2.59 (t, 2H)

### Example 44: Preparation of 3-(4-(4-(4-(3-(4-morpholinophenyl)ureido)phenyl)thieno[3,2-d]pyrimidin-7-yl)phenyl)propanoic acid

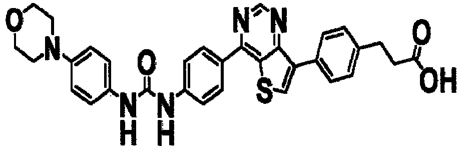

The procedure of Example 39 was repeated except for using 4-morpholinoaniline instead of 3-fluoroaniline to obtain the title compound.

¹H NMR (300 MHz, DMSO-*d*₆): *δ* 12.20 (bs, 1H), 9.29 (s, 1H), 9.07 (s, 1H), 8.72 (s, 1H), 8.59 (s, 1H), 8.18 (d, 2H), 8.00 (d, 2H), 7.74 (d, 2H), 7.37 (t, 4H), 6.91 (d, 2H), 3.73 (m, 4H), 3.03 (m, 4H), 2.89 (t, 2H), 2.60 (t, 2H)

### Example 45: Preparation of 3-(4-(4-(4-(3-(2,3,5-trifluorophenyl)ureido)phenyl)thieno[3,2-d]pyrimidin-7-yl)phenyl)propanoic acid

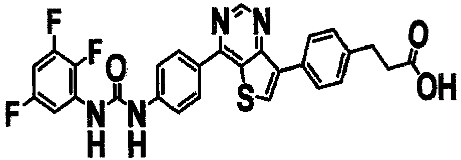

The procedure of Example 39 was repeated except for using 2,3,5-trifluoroaniline instead of 3-fluoroaniline to obtain the title compound.
¹H NMR (300 MHz, DMSO-*d*₆): *δ* 12.18 (bs, 1H), 9.58 (s, 1H), 9.28 (s, 1H), 9.13 (s, 1H), 8.71 (s, 1H), 8.20 (d, 2H), 7.98 (d, 2H), 7.90 (m, 1H), 7.74 (d, 2H), 7.35 (d, 2H), 7.13 (m, 1H), 2.86 (t, 2H), 2.56 (t, 2H)

### Example 46: Preparation of 3-(4-(4-(4-(3-(2-chloro-5-methoxyphenyl)ureido)phenyl)thieno[3,2-d]pyrimidin-7-yl)phenyl)propanoic acid

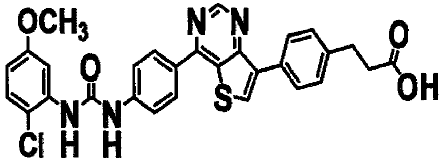

The procedure of Example 39 was repeated except for using 2-chloro-5-methoxyphenylaniline instead of 3-fluoroaniline to obtain the title compound.
¹H NMR (300 MHz, DMSO-*d*₆): *δ* 12.14 (bs, 1H), 9.86 (s, 1H), 9.29 (s, 1H), 8.73 (s, 1H), 8.41 (s, 1H), 8.22 (d, 2H), 8.00 (d, 2H), 7.89 (d, 1H), 7.76 (d, 2H), 7.35 (m, 3H), 6.64 (dd, 1H), 3.74 (s, 3H), 2.88 (t, 2H), 2.58 (t, 2H)

### Example 47: Preparation of 3-(4-(4-(4-(3-(2-fluorophenyl)ureido)phenyl)thieno[3,2-d]pyrimidin-7-yl)phenyl)propanoic acid

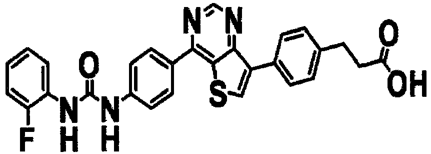

The procedure of Example 39 was repeated except for using 2-fluoroaniline instead of 3-fluoroaniline to obtain the title compound.
¹H NMR (300 MHz, DMSO-*d*₆): *δ* 12.16 (bs, 1H), 9.49 (s, 1H), 9.31 (s, 1H), 8.74 (s, 1H), 8.71 (s, 1H), 8.22 (d, 2H), 8.16 (m, 1H), 8.01 (d, 2H), 7.76 (d, 2H), 7.38 (d, 2H), 7.27 (m, 1H), 7.17 (t, 1H), 7.05 (m, 1H), 2.90 (t, 2H), 2.60 (t, 2H)

### Example 48: Preparation of 3-(4-(4-(4-(3-(4-(1H-pyrrolo-1-yl)phenyl)ureido)phenyl)thieno[3,2-d]pyrimidin-7-yl)phenyl)propanoic acid

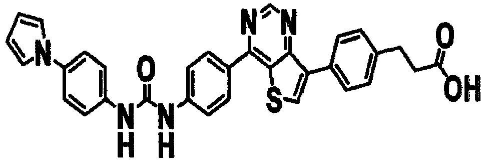

The procedure of Example 39 was repeated except for using 4-(1*H-*pyrrol-1-yl)aniline instead of 3-fluoroaniline to obtain the title compound.
¹H NMR (300 MHz, DMSO-*d*₆): *δ* 12.10 (bs, 1H), 9.28 (s, 2H), 9.07 (s, 1H), 8.71 (s, 1H), 8.18 (d, 2H), 8.00 (d, 2H), 7.76 (d, 2H), 7.56 (d, 2H), 7.48 (d, 2H), 7.38 (d, 2H), 7.28 (m, 2H), 6.22 (m, 2H), 2.89 (t, 2H), 2.58 (t, 2H)

### Example 49: Preparation of 3-(4-(4-(4-(3-(4-(2H-tetrazol-5-yl)phenyl)ureido)phenyl)thieno[3,2-d]pyrimidin-7-yl)phenyl)propanoic acid

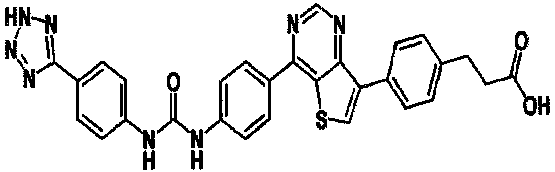

The procedure of Example 39 was repeated except for using 4-(2*H-*tetrazol-5-yl)aniline instead of 3-fluoroaniline to obtain the title compound.
¹H NMR (300 MHz, DMSO-*d*₆): *δ* 12.10 (bs, 1H), 9.31 (s, 2H), 9.22 (s, 1H), 8.74 (s, 1H), 8.21 (d, 2H), 7.99 (m, 4H), 7.78 (d, 2H), 7.68 (d, 2H), 7.39 (d, 2H), 2.89 (t, 2H), 2.60 (t, 2H)

### Example 50: Preparation of 3-(4-(4-(4-(3-(4-(4-methylpiperazin-1-yl)phenyl)ureido)phenyl)thieno[3,2-d]pyrimidin-7-yl)phenyl)propanoic acid

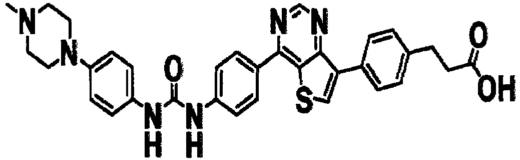

The procedure of Example 39 was repeated except for using 4-(4-methylpiperazin-1-yl)aniline instead of 3-fluoroaniline to obtain the title compound.
¹H NMR (300 MHz, DMSO-*d*₆): *δ* 12.10 (bs, 1H), 9.28 (s, 1H), 9.00 (s, 1H), 8.71 (s, 1H), 8.55 (s, 1H), 8.18 (d, 2H), 8.00 (d, 2H), 7.73 (d, 2H), 7.34 (m, 4H), 6.88 (d, 2H), 3.30 (s, 3H), 3.05 (m, 4H), 2.88 (t, 2H), 2.58 (t, 2H), 2.21 (m, 4H)

### Example 51: Preparation of 3-(4-(4-(4-(3-(1-methylpiperidin-4-yl)ureido)phenyl)thieno[3,2-d]pyrimidin-7-yl)phenyl)propanoic acid

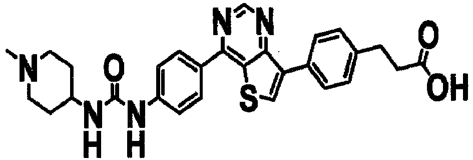

The procedure of Example 39 was repeated except for using 1-methyl-4-amino-piperidine instead of 3-fluoroaniline to obtain the title compound.
¹H NMR (300 MHz, DMSO-*d*₆): *δ* 9.90 (bs, 1H), 9.27 (s, 1H), 9.02 (s, 1H), 8.71 (s, 1H), 8.13 (d, 2H), 7.99 (d, 2H), 7.67 (d, 2H), 7.36 (d, 2H), 6.73 (d, 1H), 3.80 (m, 1H), 3.40 (m, 2H), 3.02 (m, 2H), 2.88 (t, 2H), 2.72 (d, 3H), 2.58 (t, 2H), 2.05 (m, 2H), 1.65 (m, 2H)

### Example 52: Preparation of 3-(4-(4-(4-(3-(4-(pyrrolidin-1-yl)phenyl)ureido)phenyl)thieno[3,2-d]pyrimidin-7-yl)phenyl)prapanoic acid

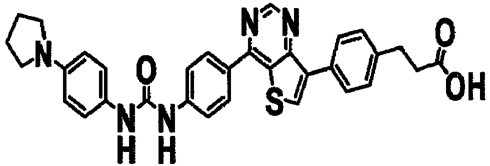

The procedure of Example 39 was repeated except for using 4-(pyrrolidin-1-yl)aniline instead of3-fluoroaniline to obtain the title compound.
¹H NMR (300 MHz, DMSO-*d*₆): *δ* 12.10 (bs, 1H), 9.27 (s, 1H), 8.99 (s, 1H), 8.71 (s, 1H), 8.44 (s, 1H), 8.16 (d, 2H), 8.99 (d, 2H), 7.71 (d, 2H), 7.36 (d, 2H), 7.25 (d, 2H), 6.49 (d, 2H), 3.17 (m, 4H), 2.88 (t, 2H), 2.58 (t, 2H), 1.93 (m, 4H)

### Example 53: Preparation of 3-(4-(4-(4-(3-(4-(4H-1,2,4-triazol-4-yl)phenyl)ureido)phenyl)thieno[3,2-d]pyrimidin-7-yl)phenyl)propanoic acid

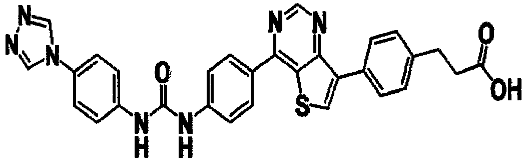

The procedure of Example 39 was repeated except for using 4-(4*H-*1,2,4-triazol-4-yl)aniline instead of3-fluoroaniline to obtain the title compound.
¹H NMR (300 MHz, DMSO-*d*₆): *δ* 12.20 (bs, 1H), 9.85 (s, 2H), 9.26 (s, 1H), 9.20 (s, 1H), 8.68 (s, 1H), 8.20 (s, 1H), 8.15 (d, 2H), 8.00 (d, 2H), 7.73 (m, 6H), 7.40 (d, 2H), 2.92 (t, 2H), 2.58 (t, 2H)

### Example 54: Preparation of 3-(4-(4-(4-(3-(4-(oxazol-2-yl)phenyl)ureido)phenyl)thieno[3,2-d]pyrimidin-7-yl)phenyl)propanoic acid

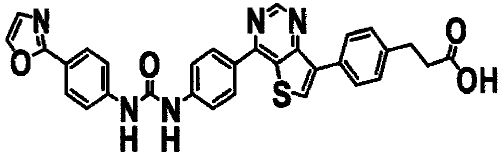

The procedure of Example 39 was repeated except for using 4-(oxazol-2-yl)aniline instead of 3-fluoroaniline to obtain the title compound.
¹H NMR (300 MHz, DMSO-*d*₆): *δ* 12.20 (bs, 1H), 9.35 (s, 1H), 9.31 (s, 2H), 8.74 (s, 1H), 8.21 (d, 2H), 8.17 (s, 1H), 8.02 (d, 2H), 7.94 (d, 2H), 7.79 (d, 2H), 7.67 (d, 2H), 7.40 (d, 2H), 7.34 (s, 1H), 2.90 (t, 2H), 2.60 (t, 2H)

### Example 55: Preparation of 3-(4-(4-(4-(3-(2-fluoro-3-(trifluoromethyl)phenyl)ureido)phenyl)thieno[3,2-d]pyrimidin-7-yl)phenyl)propanoic acid

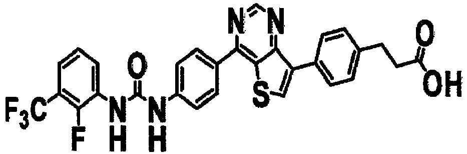

The procedure of Example 39 was repeated except for using 2-fluoro-3-(trifluoromethyl)aniline instead of 3-fluoroaniline to obtain the title compound.
¹H NMR (300 MHz, DMSO-*d*₆): *δ* 12.17 (bs, 1H), 9.57 (s, 1H), 9.31 (s, 1H), 9.01 (s, 1H), 8.74 (s, 1H), 8.46 (m, 1H), 8.23 (d, 2H), 8.01 (d, 2H), 7.77 (d, 2H), 7.38 (d, 4H), 2.89 (t, 2H), 2.60 (t, 2H)

### Example 56: Preparation of 3-(4-(4-(4-(3-(4-fluoro-2-(trifluoromethyl)phenyl)ureido)phenyl)thieno[3,2-d]pyrimidin-7-yl)phenyl)propanoic acid

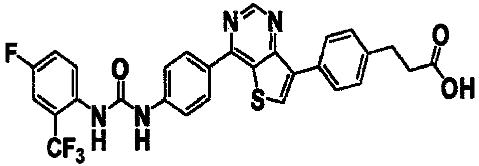

The procedure of Example 39 was repeated except for using 4-fluoro-2-(trifluoromethyl)aniline instead of 3-fluoroaniline to obtain the title compound.
¹H NMR (300 MHz, DMSO-*d*₆): *δ* 12.10 (bs, 1H), 9.76 (s, 1H), 9.30 (s, 1H), 8.73 (s, 1H), 8.35 (s, 1H), 8.20 (d, 2H), 8.00 (d, 2H), 7.90 (m, 1H), 7.75 (d, 2H), 7.61 (m, 2H), 7.38 (d, 2H) 2.89 (t, 2H), 2.59 (t, 2H)

### Example 57: Preparation of 3-(4-(4-(4-(3-(2-chlorophenyl)ureido)phenyl)-5-methyl-5H-pyrrolo[3,2-d]pyrimidin-7-phenyl)propanoic acid

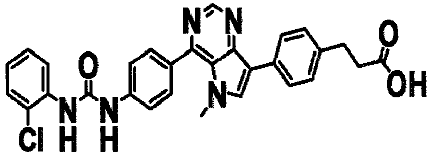

The procedure of Example 13 was repeated except for using 7-bromo-4-chloro-5-methyl-5H-pyrrolo[3,2-*d*]pyrimidine obtained in Preparation Example 3 instead of 7-bromo-4-chlorothieno[3,2-*d*]pyrimidine to obtain the title compound.
¹H NMR (300 MHz, DMSO-*d*₆): *δ* 8.94(s, 1H), 8.45(s, 1H), 8.36(s, 1H), 8.18(d, 1H), 8.12(d, 2H), 7.65(m, 4H) 7.47(d, 1H), 7.30(d, 3H), 7.06(m 1H), 3.59(s, 3H), 2.85(t, 2H), 2.59(t, 2H)

### Example 58: Preparation of 1-(4-(4-(4-(3-(3-chlorophenyl)ureido) phenyl)thieno[3,2-d]pyrimidin-7-yl)phenyl)cyclopropancarboxylic acid

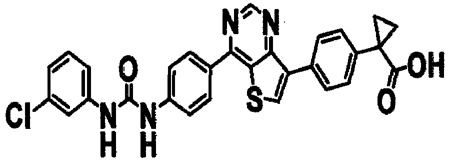

### Step 1: Preparation of 1-(4-bromophenyl)cyclopropancarbonitrile

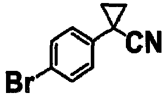

2-(4-bromophenyl)acetonitrile (1 g) was dissolved in an aqueous solution of sodium hydroxide (1.3 mL, 50% w/w), and 1,2-dibromoethane (0.7 mL) and then benzyltriethylammonium chloride (BTAC, 35 mg) were added thereto. The mixture was heated to 60°C, and stirred overnight. Upon completion of the reaction, the reaction mixture was diluted with ethyl acetate, and then washed with water. The organic layer was sequentially washed with 1N HCl and water, dried over anhydrous sodium sulfate, filtered and distilled under reduced pressure, followed by recrystallization by using diethyl ether and n-hexane to obtain the title compound (780 mg) as a solid compound.
¹H NMR (300 MHz, DMSO-*d*₆): *δ* 8.94(s, 1H), 8.45(s, 1H), 8.36(s, 1H), 8.18(d, 1H), 8.12(d, 2H), 7.65(m, 4H) 7.47(d, 1H), 7.30(d, 3H), 7.06(m 1H), 3.59(s, 3H), 2.85(t, 2H), 2.59(t, 2H)7.58(d, 2H), 7.28(d, 2H), 1.76(q, 2H), 1.52(q,2H)

### Step 2: Preparation of 1-(4-bromophenyl)cyclopropancarboxylic acid

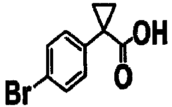

1-(4-bromophenyl)cyclopropancarbonitrile (256 mg) prepared in Step 1 was dissolved in a mixed solution of ethanol (2.5 mL) and an aqueous solution of sodium hydroxide (1.2 mL, 25% w/w). The mixture was heated to 100°C, and stirred overnight. Upon completion of the reaction, ice was added to the reaction mixture, followed by addition of dichloromethane and 1N aqueous solution of hydrochloric acid. The organic layer thus formed was separated, dried over anhydrous sodium sulfate, filtered and distilled under a reduced pressure to obtain the target compound as oil (330 mg).
¹H NMR (300 MHz, DMSO-*d*₆): *δ* 12.38 (s, 1H), 7.46(d, 2H), 7.26(d, 2H), 1.42(q, 2H), 1.11 (q, 2H)

### Step 3: Preparation of methyl 1-(4-bromophenyl)cyclopropancarboxylate

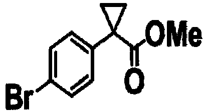

1-(4-bromophenyl)cyclopropancarboxylic acid (1.42 mg) prepared in Step 2 was dissolved in methanol (7 mL), chlorotrimethylsilane (0.2 mL) was added at room temperature and the mixture was stirred overnight. Upon completion of the reaction, the solvent was distilled to obtain the title compound as oil (200 mg).
¹H NMR (300 MHz, DMSO-*d*₆): *δ* 7.54(d, 2H), 7.34(d, 2H), 3.60(s, 3H), 1.53(d, 2H), 1.25(d, 2H)

### Step 4: Preparation of methyl 1-(4-(4,4,5,5-tetramethyl-1,3,2-dioxaborolan-2-yl)phenyl)cyclopropancarboxylate

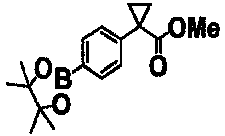

A solution was prepared by dissolving methyl 1-(4-bromophenyl)cyclopropancarboxylate (200 mg) prepared in Step 3 in 1,4-dioxane (2 mL), and tricyclohexylphosphine (26 mg), Pd₂(dba)₃ (36 mg), bis(pinacolato)diborane (220 mg, 0.862 mmol) and calcium acetate (115 mg) were added thereto. The mixture was placed under argon gas atmosphere to allow substitution reaction, heated to 100°C, and stirred overnight. Upon completion of the reaction, the reaction mixture was filtered through Celite, and washed with sodium bicarbonate solution and water.

The organic layer thus formed was dried over anhydrous sodium sulfate and filtered. The organic layer was distilled under reduced pressure to obtain the title compound (330 mg).
¹H NMR (300 MHz, DMSO-*d*₆): *δ* 7.60(d, 2H), 7.32(d, 2H), 3.52(s, 3H), 1.48(t, 2H), 1.27(s, 12H)

### Step 5 : Preparation of 1-(4-(4-(4-(3-(3-chlorophenyl)ureido)phenyl)thieno[3,2-d]pyrimidin-7-yl)phenyl)cyclopropancarboxylic acid

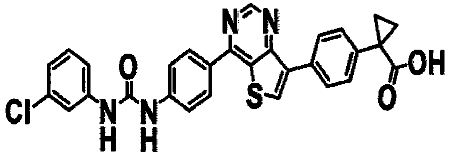

The procedure of Example 2 was repeated except for using methyl 1-(4-(4,4,5,5-tetramethyl-1,3,2-dioxaborolan-2-yl)phenyl)cyclopropancarboxylate obtained in Step 4, instead of methyl 2-(4-(4,4,5,5-tetramethyl-1,3,2-dioxaborolan-2-yl)phenyl)acetate, to obtain the title compound.
¹H NMR (300 MHz, DMSO-*d*₆): *δ* 1.2.35(s, 1H), 9.30(s, 1H), 9.18(s, 1H), 9.04(s, 1H), 8.74(s, 1H), 8.20(d, 2H), 7.99(d, 2H), 7.75(d, 3H), 7.46(d, 2H), 7.30(d, 2H), 7.00(m, 1H), 1.48(d, 2H), 1.20(d, 2H)

### Example 59: Preparation of 2-(4-(4-(4-(3-(3-chlorophenyl)ureido)phenyl)thieno[3,2-d]pyrimidin-7-yl)phenyl)-2-methylpropanoic acid

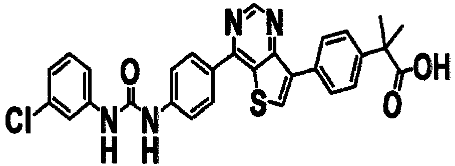

### Step 1: Preparation of methyl 2-(4-bromophenyl)propanoate

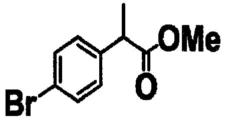

Methyl 2-(4-bromophenyl)acetate (220 mg) prepared in Step 1 of Example 1 was dissolved in dimethylformamide (3 mL), NaH (53 mg) was added thereto, and the mixture was stirred for 30 mins at room temperature. Iodomethane (0.14 mL) was added to the mixture which was heated to 50°C, followed by stirring for 2 hrs. The reaction mixture was cooled down to room temperature, and water and ethyl acetate were added to form an organic and an aqueous layer. The organic layer thus obtained was dried over anhydrous sodium sulfate, filtered and distilled under reduced pressure, and purified by Prep. TLC (n-Hex:EA= 1:1) to obtain the title compound (200 mg).
¹H NMR (300 MHz, DMSO-*d*₆): *δ* 7.50 (d, 2H), 7.27(d, 2H), 3.81(q, 1H), 3.58(s, 3H), 1.37(d, 3H)

### Step 2: Preparation of methyl 2-(4-bromophenyl)-2-methylpropanoate

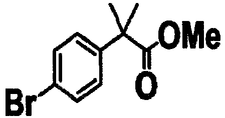

Methyl 2-(4-bromophenyl)propanoate (100 mg) prepared in Step 1 was dissolved in tetrahydrofuran (2 mL), and the mixture was cooled down to -78°C. LHMDS (1M in toluene, 0.09 mL) was added at -78°C, and the mixture was further stirred for 30 mins. A solution formed by adding iodomethane (0.10 mL) in tetrahydrofuran (2 mL) was added dropwise thereto, followed by stirring for 30 mins. The mixture was heated to room temperature, stirred for 1 hr, cooled down to 0°C, and t-BuOK (1 M in THF) was added dropwise to the mixture. The mixture was stirred for 30 mins, heated to room temperature, and further stirred for 1 hr. Upon completion of the reaction, ice was added to the reaction mixture, and ethyl acetate and an aqueous solution of ammonium chloride were added to form an organic and an aqueous layer. The organic layer thus obtained was dried over anhydrous sodium sulfate, filtered and distilled under reduced pressure, and purified by Prep.TLC (n-Hex:EA = 9:1) to obtain the title compound (80 mg).
¹H NMR (300 MHz, DMSO-*d*₆): *δ* 7.52(d, 2H), 7.27(d, 2H), 3.58(s, 3H), 1.48(s, 6H)

### Step 3: Preparation of methyl 2-methyl-2-(4-(4,4,5,5-tetramethyl-1,3,2-dioxaborolan-2-yl)phenyl)propanoate

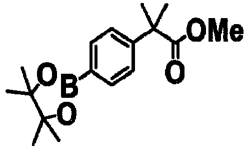

The procedure of Step 4 in Example 58 was repeated except for using methyl 2-(4-bromophenyl)-2-methylpropanoate obtained in Step 2, instead of methyl 1-(4-bromophenyl)-2-methylpropanoate, to obtain the title compound.
¹H NMR (300 MHz, DMSO-*d*₆): *δ* 7.45(d, 2H), 7.26(d, 2H), 3.56(s, 3H), 1.36(s, 6H), 1.27(s, 12H)

### Step 4: Preparation of 2-(4-(4-(4-(3-(3-chlorophenyl)ureido)phenyl)thieno[3,2-d]pyrimidin-7-yl)phenyl)-2-methylpropanoic acid

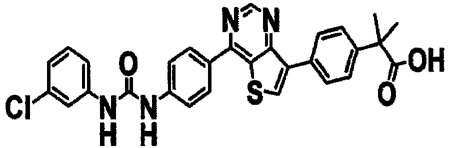

The procedure of Step 5 in Example 58 was repeated except for using methyl 2-methyl-2-(4-(4,4,5,5-tetramethyl-1,3,2-dioxaborolan-2-yl)phenyl) propanoate obtained in Step 3, instead of methyl 1-(4-(4,4,5,5-tetramethyl-1,3,2-dioxaborolan-2-yl)phenyl)cyclopropancarboxylate, to obtain the title compound.
¹H NMR (300 MHz, DMSO-*d*₆): *δ* 9.28(s, 1H), 9.11(s, 1H), 9.02(s, 1H), 8.75(s, 1H), 8.20(d, 2H), 8.03(d, 2H), 7.75(dd, 3H), 7.46(d, 2H), 7.30(m, 2H), 7.02(m, 1H), 1.55(d, 6H)

### Example 60: Preparation of 2-(4-(4-(4-(pyrimidin-2-ylamino)phenyl)thieno[3,2-d]pyrimidin-7-yl)phenyl)acetic acid

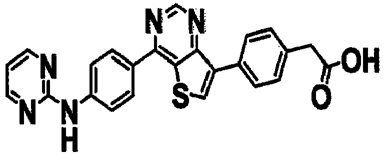

Methyl 2-(4-(4-(4-(*tert*-butoxycarbonylamino)phenyl)thieno[3,2-*d*]pyrimidin-7-yl)phenyl)acetate (150 mg) obtained in Step 4 of Example 1 was introduced to dichloromethane (2 mL), trifluoroacetic acid (0.8 mL) was added thereto and the mixture was stirred for 2 hrs at room temperature. The mixture thus obtained was distilled under reduced pressure to remove the solvent. Dichloromethane and an aqueous solution of sodium bicarbonate were added thereto, stirred sufficiently, and the organic layer thus formed was extracted. The organic layer was dried over anhydrous sodium sulfate, distilled under reduced pressure. The residue was mixed with 1,4-dioxane (2.5 mL), and 2-chloropyrimidine (45 mg), xantphos (30 mg), Pd(OAc)₂ (5 mg) and cesium carbonate (170 mg) were added thereto, followed by stirring overnight at 100°C. The reaction mixture was cooled down to room temperature, diluted with dichloromethane, and the organic layer was washed with sodium bicarbonate solution. The reactant was dried over anhydrous sodium sulfate. After removing the solvent, the reactant was crystallized by using a mixed solvent of dichloromethane and ethyl acetate. The crystal thus obtained was added to a mixed solution of tetrahydrofuran, methanol and water (tetrahydrofuran:methanol:water = 1:1:1), and was stirred. Sodium hydroxide (13 mg) was added thereto and the mixture was further stirred. The resulting mixture was distilled under reduced pressure to remove the solvent, and the pH was adjusted to the range of 3 to 4 by using 1N HCl. A solid thus obtained was filtered and washed with water to obtain the title compound (11 mg).
¹H NMR (300 MHz, DMSO-*d*₆): *δ* 12.50 (bs, 1H), 10.13 (s, 1H), 9.30 (s, 1H), 8.76 (s, 1H), 8.58 (d, 2H), 8.21 (d, 2H), 8.06 (m, 4H), 7.42 (d, 2H), 6.96 (t, 1H), 3.65 (s, 2H)

### Example 61: Preparation of 2-(4-(4-(4-(benzo[d]thiazol-2-ylamino)phenyl)thieno[3,2-d]pyrimidin-7-yl)phenyl)acetic acid

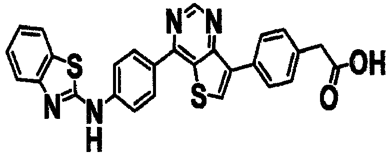

The procedure of Example 60 was repeated except for using 2-chlorobenzothiazole, instead of 2-chloropyrimidine, to obtain the title compound.
¹H NMR (300 MHz, DMSO-*d*₆): *δ* 12.32 (bs, 1H), 10.95 (s, 1H), 9.31 (s, 1H), 8.77 (s, 1H), 8.28 (d, 2H), 8.07 (m, 4H), 7.86 (d, 1H), 7.70 (d, 1H), 7.37 (m, 3H), 7.21 (t, 1H), 3.73 (s, 2H)

### Example 62: Preparation of 2-(4-(4-(4-(6-chlorobenzo[d]thiazol-2-ylamino)phenyl)thieno[3,2-d]pyrimidin-7-yl)phenyl)acetic acid

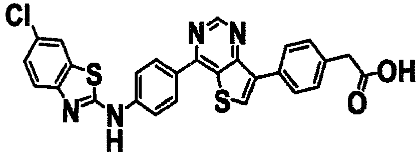

The procedure of Example 60 was repeated except for using 6-chloro-2-bromobenzothiazole, instead of 2-chloropyrimidine, to obtain the title compound.
¹H NMR (300 MHz, DMSO-*d*₆): *δ* 12.40 (bs, 1H), 11.01 (s, 1H), 9.31 (s, 1H), 8.77 (s, 1H), 8.27 (d, 2H), 8.03 (m, 5H), 7.67 (d, 1H), 7.39 (m, 3H), 3.64 (s, 2H)

### Example 63: Preparation of 3-(4-(4-(3-(3-chlorophenyl)ureido)thieno[3,2-d]pyrimidin-7-yl)phenyl)propanoic acid

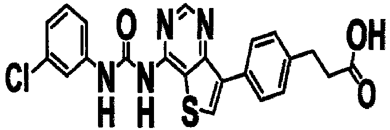

### Step 1: Preparation of methyl 3-(4-(4-methylthio)thieno[3,2-d]pyrimidin-7-yl)phenyl)propanoate

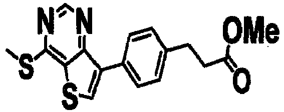

7-bromo-4-methylsulfonylthieno[3,2-*d*]pyrimidine (150 mg) prepared in Preparation Example 2, tetrakis tiphenylphosphine palladium (34g), 2N aqueous solution of sodium carbonate (0.9 mL) and methyl 3-(4-(4,4,5,5-tetramethyl-1,3,2-dioxaborolan-2-yl)phenyl)acetate (217 mg) used in Example 7 were mixed and stirred overnight at 100°C. The reaction mixture was cooled down to room temperature, diluted in dichloromethane, and washed with an aqueous solution of sodium bicarbonate. The resulting compound was dried over anhydrous sodium sulfate, distilled under reduced pressure and purified by column chromatography to obtain the title compound (190 g) as a solid compound.
¹H NMR (300 MHz, DMSO-*d*₆): *δ* 9.14 (s, 1H), 8.78 (s, 1H), 7.97 (d, 2H), 7.37 (d, 2H), 3.59 (s, 3H), 2.92 (t, 2H), 2.69 (t, 2H)

### Step 2: Preparation of methyl 3-(4-(4-chlorothieno[3,2-d]pyrimidin-7-yl)]phenyl)propanoate

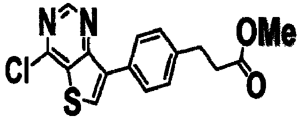

Methyl 3-(4-(4-methylthio)thieno[3,2-*d*]pyrimidin-7-yl)phenyl) propanoate (216 mg) prepared in Step 1 was diluted in acetonitrile (13 mL), sulfuryl fluoride (423 mg) was slowly added thereto at 0°C, and stirred for 1 hr. Dichloromethane (16 mL) was added to the mixture, which was washed with an aqueous solution of sodium bicarbonate and dried over anhydrous sodium sulfate. The solvent was removed by subjecting the residue thus obtained to distillation under reduced pressure to obtain the title compound (230 mg) as a solid compound.
¹H NMR (300 MHz, DMSO-*d*₆): *δ* 9.14 (s, 1H), 8.78 (s, 1H), 7.97 (d, 2H), 7.37 (d, 2H), 3.59 (s, 3H), 2.92 (t, 2H), 2.69 (t, 2H)

### Step 3: Preparation of methyl 3-(4-(4-aminothieno[3,2-d]pyrimidin-7-yl)phenyl)propanoate

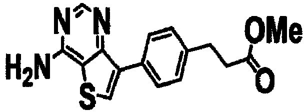

Methyl 3-(4-(4-chlorothieno[3,2-*d*]pyrimidin-7-yl)phenyl)propanoate (230 mg) prepared in Step 2 was added to a 2 M ammonia isopropylalcohol solution (5 mL), and the mixture was stirred overnight at 100°C. The reaction mixture was cooled down to room temperature, distilled under reduced pressure to remove the solvent, diluted with ethyl acetate, and washed with an aqueous solution of sodium bicarbonate. The reactant was dried over anhydrous sodium sulfate, distilled under reduced pressure to remove the solvent, and recrystallized by using diethyl ether and n-hexane to obtain the title compound (150 mg) as a solid compound.
¹H NMR (300 MHz, DMSO-*d*₆): *δ* 8.45 (s, 1H), 8.30 (s, 1H), 7.96 (d, 2H), 7.49 (s, 2H), 7.31 (d, 2H), 3.59 (s, 3H), 2.89 (t, 2H), 2.67 (t, 2H)

### Step 4: Preparation of 3-(4-(4-(3-(3-chlorophenyl)ureido)thieno[3,2-d]pyrimidin-7-yl)phenyl)propanoic acid

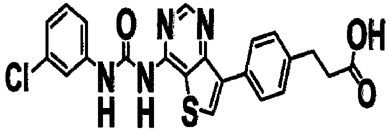

Methyl 3-(4-(4-aminothieno[3,2-*d*]pyrimidin-7-yl)phenyl)propanoate (150 mg) prepared in Step 3 was dissolved in tetrahydrofuran, 3-chlorophenylisocyanate (80 mg) was added thereto, and the mixture was stirred for 24 hrs at room temperature. The reaction solvent was concentrated, and diethyl ether was added thereto for crystallization. The resulting compound was filtered and the filtrate thus obtained was added to a mixed solution of tetrahydrofuran, methanol and water (tetrahydrofuran:methanol:water = 1:1:1), and the mixture was stirred. Sodium hydroxide (19.2 mg) was added thereto, and the mixture was further stirred for 2 hrs. The resulting mixture was distilled under reduced pressure to remove the solvent, and the pH was adjusted to the range of 3 to 4 by using 1N HCl. A solid thus obtained was filtered and washed with water to obtain the title compound (180 g).
¹H NMR (300 MHz, DMSO-*d*₆): *δ* 11.66 (bs, 1H), 8.86 (s, 1H), 8.49 (s, 1H), 7.90 (d, 2H), 7.86 (s, 1H), 7.53 (d, 1H), 7.36 (m, 3H), 7.11 (d, 1H), 2.88 (t, 2H), 2.53 (t, 2H)

### Example 64: Preparation of 2-(4-(4-(3-(3-chlorophenyl)ureido)thieno[3,2-d]pyrimidin-7-yl)benzoamido)acetic acid

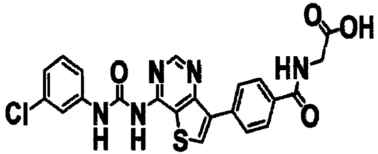

### Step 1: Preparation of 4-(4-(3-(3-chlorophenyl)ureido)thieno[3,2-d]pyrimidin-7-yl)benzoic acid

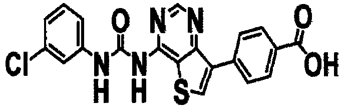

The procedure of Example 63 was repeated except for using methyl 4-(4,4,5,5-tetramethyl-1,3,2-dioxaborolan-2-yl)benzoate, instead of methyl 3-(4-(4,4,5,5-tetramethyl-1,3,2-dioxaborolan-2-yl)phenyl)propanoate, to obtain the title compound.

### Step 2: Preparation of 2-(4-(4-(3-(3-chlorophenyl)ureido-thieno[3,2-d]pyrimidin-7-yl)benzoamido)acetic acid

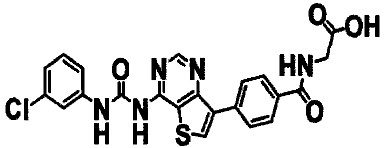

4-(4-(3-(3-chlorophenyl)ureido)thieno[3,2-*d*]pyrimidin-7-yl)benzoic acid (380 mg) prepared in Step 1 was dissolved in dimethylformamide (2.5 mL). Glycine ethyl ester (127 mg), 2-(7-aza-1*H-*benzotriazol-1-yl)-1,1,3,3-tetramethyluronium hexafluorophosphate (1.04 g) and *N,N-*diisopropylethylamine (0.8 mL) were added thereto, and the mixture was stirred under reflux overnight at 40°C. Upon completion of the reaction, ethyl acetate and distilled water were added to the reaction mixture to form organic and aqueous layers. The organic layer thus obtained was dried over anhydrous sodium sulfate, filtered and concentrated, and purified by chromatography. The intermediate thus obtained was added to a mixed solution (8 mL) of tetrahydrofuran, methanol and water (tetrahydrofuran:methanol:water = 1:1:1). Sodium hydroxide (108 mg) was added to the mixture, which was stirred for 2 hrs at room temperature. The resulting mixture was distilled under reduced pressure to remove the solvent, and 1N HCl was added to adjust the pH to the range of 3 to 4. A solid thus obtained was filtered and washed with water to obtain the title compound (320 g).
¹H NMR (300 MHz, DMSO-*d*₆): *δ* 12.10 (bs, 1H), 11.21 (s, 1H), 10.55 (s, 1H), 8.94 (s, 1H), 8.88 (t, 1H), 8.75 (s, 1H), 8.18 (d, 2H), 7.98 (d, 2H), 7.83 (s, 1H), 7.50 (d, 1H), 7.37 (t, 1H), 7.14 (d, 1H), 3.93 (d, 2H)

### Example 65: Preparation of (S)-2-(4-(4-(3-(3-chlorophenyl)ureido)thieno[3,2-d]pyrimidin-7-yl)benzoamido)-3-methylbutanoic acid

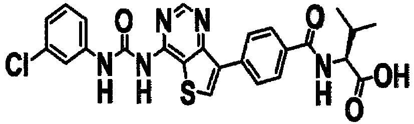

The procedure of Example 64 was repeated except for using L-valine methyl ester hydrochloride, instead of glycine ethyl ester, in Step 2 to obtain the title compound.
¹H NMR (300 MHz, DMSO-*d*₆): *δ* 12.65 (bs, 1H), 11.23 (s, 1H), 10.62(s, 1H), 8.96 (s, 1H), 8.74 (s, 1H), 8.46 (d, 1H), 8.16 (d, 2H), 8.00 (d, 2H), 7.80 (s, 1H), 7.51 (m, 1H), 7.38 (t, 1H), 7.15 (m, 1H), 4.32 (t, 1H), 2.19 (t, 1H), 0.97 (t, 6H)

### Experimental Example 1: Assay for Inhibition of DGAT1 Activity (IC₅₀)

In order to identify DGAT1 inhibitors, *in vitro* enzymatic assays were performed by using homogenates of HepG2 cell line (ATCC, USA, HB-8065). Briefly stated, HepG2 cells were inoculated in 10% FBS-supplemented medium at 1.0 x 10⁷ cells/well, cultured at 37°C, and harvested 72 hrs later. The harvested cells were lysed by ultrasonic treatment, and centrifuged for at 4°C (2000 x g, 10 mins) to remove any uncracked cells and impurities. The supernatant thus formed was obtained, and stored at -70°C after its protein contents were quantitatively analyzed.

DGAT1 activation was assayed by a modification of the method described by Coleman (Methods in Enzymology, 1992, 209, 98-102). The compound of the present invention (0.01 to 100 mM) was incubated with HepG2 protein (25 µg), MgCl₂ (150 mM) and 1,2-dioleoyl-sn-glycerol (30 µM) in a total assay volume of 200 µL in plastic tubes.

The reaction was started by adding ¹⁴C oleoyl coenzyme A (10 µM final concentration) and incubated at room temperature for 60 mins. The reaction was ended when a mixed solution (300 µL) of 2-propanol and heptane (7:1) was added thereto. Radioactive triolein product was separated into the organic phase by adding heptane (200 µL) and 1.0 M carbonate buffer (200 µL, pH 9.5). DGAT 1 activity was quantified by counting aliquots of the upper heptane layer by liquid scintigraphy. The measured inhibitory activities of the inventive compounds against DGAT1 in HepG2 cells, i.e., IC₅₀ values, are shown in Table 1 below.

**[Table 1]**

| Example | IC₅₀ | Example | IC₅₀ | Example | IC₅₀ |
|---|---|---|---|---|---|
| 1 | < 5 nM | 24 | < 5 nM | 46 | < 5 nM |
| 2 | < 1 nM | 28 | < 5 nM | 49 | < 1 nM |
| 3 | < 1 nM | 30 | < 1 nM | 50 | < 20 nM |
| 4 | < 1 nM | 31 | < 10 nM | 51 | < 1 nM |
| 7 | < 1 nM | 34 | < 10 nM | 52 | < 1 nM |
| 14 | < 5 nM | 35 | < 10 nM | 53 | < 50 nM |
| 15 | < 5 nM | 39 | < 1 nM | 55 | < 5 nM |
| 16 | < 1 nM | 40 | < 1 nM | 57 | < 50 nM |
| 20 | < 5 nM | 42 | < 5 nM | 59 | < 1 nM |
| 21 | < 1 nM | 44 | < 10 nM | 61 | < 5 nM |

As shown in Table 1 above, the compounds of the present invention significantly inhibited the activity of DGAT1.

Therefore, the compounds of the present invention can effectively inhibit the activity of DGAT1, and thus, may be used as a therapeutic agent for a disease or condition mediated by the activity of DGAT1 such as obesity, type II diabetes, etc.

## Claims

1. A compound of formula (I) or pharmaceutically acceptable salts thereof: 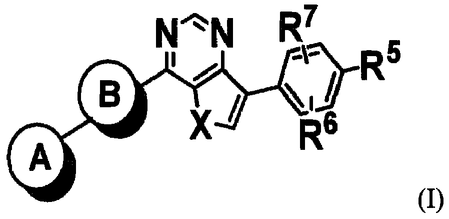
wherein X is each independently S or NR wherein R is H or C₁₋₄alkyl;
A is 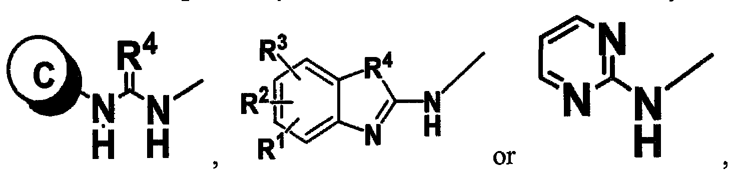 wherein C is hydrogen, C₁₋₆alkyl, C₃₋₈cycloalkyl, C₆₋₁₄aryl, C₅₋₁₃heteroaryl or C₃₁₃heterocycloalkyl, wherein the heteroaryl or heterocycloalkyl is unsubstituted or substituted with C₁₋₆alkyl, halogen, diC₁₋₆alkylamino or C₁₋₆alkoxy, and the aryl is unsubstituted or substituted with halogen, C₁₋₆alkyl, halogen-substituted C₁₋₆alkyl, C₁₋₄alkoxy, C₃₋₈cycloalkyl, C₁₋₆alkylamino, diC₁₋₆alkylamino, C₆₁₄aryl, C₅₋₁₃heteroaryl or C₃₋₁₃heterocycloalkyl (wherein the aryl, heteroaryl or heterocycloalkyl is unsubstituted or substituted with C₁₋₆alkyl, halogen, diC₁₆alkylamino or C₁₋₆alkoxy),
R⁴ is O, S or NR wherein R is H or C₁₋₄alkyl;
B is unsubstituted or substituted C₆₋₁₄aryl or heteroaryl;
R⁵ is C₁₋₆alkyl unsubstituted or substituted with a substituent selected from the group consisting of carboxy, C₁₋₄alkoxycarbonyl, aminocarboxyl and hydroxyl; C₃₋₈cycloalkyl unsubstituted or substituted with carboxy or C₁₄alkoxycarbonyl; or carboxyC₁₋₆alkylamido, C₁₋₆alkylsulfonyl, C₁₋₆alkylamino, C₁₋₆alkylamido, diC₁₋₆alkylamino, C₁₋₆alkoxy, C₁₋₆alkylcarbonyl, C₁₆alkoxycarbonyl, C₆₋₁₄aryl, C₅₋₁₃heteroaryl or C₃₋₁₃heterocycloalkyl wherein the aryl, heteroaryl or heterocycloalkyl is unsubstituted or substituted with C₁₋₆alkyl, halogen, diC₁₋₆alkylamino or C₁₋₆alkoxy; and
R⁶ and R⁷ are each independently H, halogen, C₁₋₄alkoxy unsubstituted or substituted with 1 to 3 halogens, C₃₋₈cycloalkyl, C₁₋₆alkylamino, diC₁₆alkylamino, C₆₋₁₄aryl, C₅₋₁₃heteroaryl, or C₃₋₁₃heterocycloalkyl.

2. The compound of claim 1, wherein
X is each independently S or NR wherein R is H or C₁₋₄alkyl;
A is 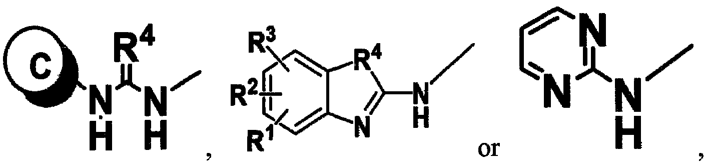 wherein C is C₃₋₈cycloalkyl, C₆₋₁₄aryl, C₅₋₁₃heteroaryl or C₃₁₃heterocycloalkyl, wherein the heteroaryl or heterocycloalkyl is unsubstituted or substituted with halogen, and the aryl is unsubstituted or substituted with halogen, C₁₋₆alkyl, C₁₋₆alkyl substituted with halogen, C₁₋₄alkoxy, C₁₆alkylamino, diC₁₋₆alkylamino, C₅₋₁₃heteroaryl or C₃₋₁₃heterocycloalkyl,
R⁴ is O or S;
B is unsubstituted or substituted C₆₋₁₄aryl;
R⁵ is C₁₋₆alkyl substituted with a substituent selected from the group consisting of carboxy, C₁₋₄alkoxycarbonyl, aminocarboxyl and hydroxyl, carboxyC₃₋₈cycloalkyl, carboxyC₁₋₆alkylamido or C₁₋₆alkylsulfonyl; and
R⁶ and R⁷ are each independently H or halogen.

3. The compound of claim 1, wherein the compound of formula (I) is selected from the group consisting of:
1) 2-(4-(4-(4-(3-phenylureido)phenyl)thieno[3,2-*d*]pyrimidin-7-yl)phenyl)acetic acid;
2) 2-(4-(4-(4-(3-(3-chlorophenyl)ureido)phenyl)thieno[3,2-*d*]pyrimidin-7-yl) phenyl)acetic acid;
3) 4-(4-(4-(3-(3-chlorophenyl)ureido)phenyl)thieno[3,2-*d*]pyrimidin-7-yl)benzoic acid;
4) 2-(4-(4-(4-(3-(3,4-difluorophenyl)ureido)phenyl)thieno[3,2-*d*]pyrimidin-7-yl)phenyl)acetic acid;
5) 2-(4-(4-(4-(3-(2-(trifluoromethoxy)phenyl)ureido)phenyl)thieno[3,2-*d*]pyrimidin-7-yl)phenyl)acetic acid;
6) 2-(4-(4-(3-(3-chlorophenyl)ureido)thieno[3,2-*d*]pyrimidin-7-yl)benzoamido)acetic acid;
7) 2-(4-(4-(4-(3-(4-methoxyphenyl)ureido)phenyl)thieno[3,2-*d*]pyrimidin-7-yl)phenyl)acetic acid;
8) (S)-2-(4-(4-(3-(3-chlorophenyl)ureido)thieno[3,2-*d*]pyrimidin-7-yl)benzoamido)-3-methylbutanoic acid;
9) 3-(4-(4-(4-(3-(3-chlorophenyl)ureido)phenyl)thieno[3,2-*d*]pyrimidin-7-yl)phenyl)propanoic acid;
10) 2-(4-(4-(4-(3-ethylureido)phenyl)thieno[3,2-*d*]pyrimidin-7-yl)phenyl)acetic acid;
11) methyl 2-(4-(4-(4-(3-(3-chlorophenyl)ureido)phenyl)thieno[3,2-*d*]pyrimidin-7-yl)phenyl)acetate;
12) 2-(4-(4-(4-(3-(2-chlorophenyl)ureido)phenyl)thieno[3,2-*d*]pyrimidin-7-yl)phenyl)acetic acid;
13) 2-(4-(4-(4-(3-(4-chlorophenyl)ureido)phenyl)thieno[3,2-*d*]pyrimidin-7-yl)phenyl)acetic acid;
14) 2-(4-(4-(4-(benzo[*d*]thiazol-2-ylamino)phenyl)thieno[3,2-*d*]pyrimidin-7-yl)phenyl)acetic acid;
15) 2-(4-(4-(4-(pyrimidin-2-ylamino)phenyl)thieno[3,2-*d*]pyrimidin-7-yl)phenyl)acetic acid;
16) 2-(4-(4-(4-(3-pyridin-3-ylureido)phenyl)thieno[3,2-*d*]pyrimidin-7-yl)phenyl)acetic acid;
17) 2-(4-(4-(4-(6-chlorobenzo[*d*]thiazol-2-ylamino)phenyl)thieno[3,2-*d*]pyrimidin-7-yl)phenyl)acetic acid;
18) 2-(4-(4-(4-(3-(2-chloropyridin-4-yl)ureido)phenyl)thieno[3,2-*d*]pyrimidin-7-yl)phenyl)acetic acid;
19) 2-(4-(4-(4-(3-(4-(dimethylamino)phenyl)ureido)phenyl)thieno[3,2-*d*]pyrimidin-7-yl)phenyl)acetic acid;
20) 2-(4-(4-(4-(3-(4-chloropyridin-2-yl)ureido)phenyl)thieno[3,2-*d*]pyrimidin-7-yl)phenyl)acetic acid;
21) 2-(4-(4-(4-(3-pyridin-2-ylureido)phenyl)thieno[3,2-*d*]pyrimidin-7-yl)phenyl)acetic acid;
22) 2-(4-(4-(4-(3-pyridin-4-ylureido)phenyl)thieno[3,2-*d*]pyrimidin-7-yl)phenyl)acetic acid;
23) 4-(4-(4-(4-(3-(3-chlorophenyl)ureido)phenyl)thieno[3,2-*d*]pyrimidin-7-yl)phenyl)butanoic acid;
24) 3-(4-(4-(4-(3-(2-chlorophenyl)ureido)phenyl)thieno[3,2-*d*]pyrimidin-7-yl)phenyl)propanoic acid;
25) 3-(4-(4-(4-(3-(3,4-difluorophenyl)ureido)phenyl)thieno[3,2-*d*]pyrimidin-7-yl)phenyl)propanoic acid;
26) 3-(4-(4-(4-(3-(4-methoxyphenyl)ureido)phenyl)thieno[3,2-*d*]pyrimidin-7-yl)phenyl)propanoic acid;
27) 3-(4-(4-(4-(3-(3-fluorophenyl)ureido)phenyl)thieno[3,2-*d*]pyrimidin-7-yl)phenyl)propanoic acid;
28) 3-(4-(4-(4-(3-(4-(dimethylamino)phenyl)ureido)phenyl)thieno[3,2-*d*]pyrimidin-7-yl)phenyl)propanoic acid;
29) 3-(4-(4-(4-(3-(2,3-difluorophenyl)ureido)phenyl)thieno[3,2-*d*]pyrimidin-7-yl)phenyl)propanoic acid;
30) 3-(4-(4-(4-(3-(4-chloropyridin-2-yl)ureido)phenyl)thieno[3,2-*d*]pyrimidin-7-yl)phenyl)propanoic acid;
31) 2-(4-(4-(4-(3-cyclohexylureido)phenyl)thieno[3,2-*d*]pyrimidin-7-yl)phenyl)acetic acid;
32) 2-(4-(4-(4-(3-pyridazin-3-ylureido)phenyl)thieno[3,2-*d*]pyrimidin-7-yl)phenyl)acetic acid;
33) 3-(4-(4-(4-(3-(2-chloro-6-methylphenyl)ureido)phenyl)thieno[3,2-*d*]pyrimidin-7-yl)phenyl)propanoic acid;
34) 3-(4-(4-(4-(3-(3-chloro-4-methoxyphenyl)ureido)phenyl)thieno[3,2-*d*]pyrimidin-7-yl)phenyl)propanoic acid;
35) 1-(4-(4-(4-(3-(3-chlorophenyl)ureido)phenyl)thieno[3,2-*d*]pyrimidin-7-yl)phenyl)cyclopropancarboxylic acid;
36) 2-(4-(4-(4-(3-(3-chlorophenyl)ureido)phenyl)thieno[3,2-*d*]pyrimidin-7-yl)phenyl)-2-methylpropanoic acid;
37) 3-(4-(4-(4-(3-(3-methoxyphenyl)ureido)phenyl)thieno[3,2-*d*]pyrimidin-7-yl)phenyl)propanoic acid;
38) 3-(4-(4-(4-(3-(2-methoxyphenyl)ureido)phenyl)thieno[3,2-*d*]pyrimidin-7-yl)phenyl)propanoic acid;
39) 3-(4-(4-(4-(3-p-tolylureido)phenyl)thieno[3,2-*d*]pyrimidin-7-yl)phenyl)propanoic acid;
40) 3-(4-(4-(4-(3-(5-fluoro-2-methylphenyl)ureido)phenyl)thieno[3,2-*d*]pyrimidin-7-yl)phenyl)propanoic acid;
41) 3-(4-(4-(4-(3-(2-fluoro-5-(trifluoromethyl)phenyl)ureido)phenyl) thieno[3,2-*d*]pyrimidin-7-yl)phenyl)propanoic acid;
42) 3-(4-(4-(4-(3-(3,4,5-trimethoxyphenyl)ureido)phenyl)thieno[3,2-*d*]pyrimidin-7-yl)phenyl)propanoic acid;
43) 3-(4-(4-(4-(3-(2,3-dichlorophenyl)ureido)phenyl)thieno[3,2-*d*]pyrimidin-7-yl)phenyl)propanoic acid;
44) 3-(4-(4-(4-(3-(3-(trifluoromethyl)phenyl)ureido)phenyl)thieno[3,2-*d*]pyrimidin-7-yl)phenyl)propanoic acid;
45) 3-(4-(4-(4-(3-(2,5-difluorophenyl)ureido)phenyl)thieno[3,2-*d*]pyrimidin-7-yl)phenyl)propanoic acid;
46) 3-(4-(4-(4-(3-(4-morpholinophenyl)ureido)phenyl)thieno[3,2-*d*]pyrimidin-7-yl)phenyl)propanoic acid;
47) 3-(4-(4-(4-(3-(2,3,5-trifluorophenyl)ureido)phenyl)thieno[3,2-*d*]pyrimidin-7-yl)phenyl)propanoic acid;
48) 3-(4-(4-(4-(3-(2-chloro-5-methoxyphenyl)ureido)phenyl)thieno[3,2-*d*]pyrimidin-7-yl)phenyl)propanoic acid;
49) 3-(4-(4-(4-(3-(2-fluorophenyl)ureido)phenyl)thieno[3,2-*d*]pyrimidin-7-yl)phenyl)propanoic acid;
50) 3-(4-(4-(4-(3-cyclohexylureido)phenyl)thieno[3,2-*d*]pyrimidin-7-yl)phenyl)propanoic acid;
51) 3-(4-(4-(4-(3-(2-chlorophenyl)ureido)phenyl)-5-methyl-5*H*-pyrrolo [3,2-*d*]pyrimidin-7-yl)phenyl)propanoic acid;
52) 3-(4-(4-(4-(3-(4-(1*H-*pyrrolo-1-yl)phenyl)ureido)phenyl)thieno[3,2-*d*]pyrimidin-7-yl)phenyl)propanoic acid;
53) 3-(4-(4-(4-(3-(4-(2*H*-tetrazol-5-yl)phenyl)ureido)phenyl)thieno[3,2-*d*]pyrimidin-7-yl)phenyl)propanoic acid;
54) 3-(4-(4-(4-(3-(4-(4-methylpiperazin-1-yl)phenyl)ureido)phenyl) thieno[3,2-*d*]pyrimidin-7-yl)phenyl)propanoic acid;
55) 3-(4-(4-(4-(3-(1-methylpiperidin-4-yl)ureido)phenyl)thieno[3,2-*d*]pyrimidin-7-yl)phenyl)propanoic acid;
56) 3-(4-(4-(4-(3-(4-(pyrrolidin-1-yl)phenyl)ureido)phenyl)thieno[3,2-*d*]pyrimidin-7-yl)phenyl)propanoic acid;
57) 3-(4-(4-(4-(3-(3-chlorophenyl)ureido)phenyl)thieno[3,2-*d*]pyrimidin-7-yl)phenyl)propanamide;
58) 1-(3-chlorophenyl)-3-(4-(7-(4-(3-hydroxypropyl)phenyl)thieno[3,2-*d*]pyrimidin-4-yl)phenyl)urea;
59) 3-(4-(4-(4-(3-(4-(4*H*-1,2,4-triazol-4-yl)phenyl)ureido)phenyl)thieno [3,2-*d*]pyrimidin-7-yl)phenyl)propanoic acid;
60) 3-(4-(4-(4-(3-(4-(oxazol-2-yl)phenyl)ureido)phenyl)thieno[3,2-*d*]pyrimidin-7-yl)phenyl)propanoic acid;
61) 3-(4-(4-(3-(3-chlorophenyl)ureido)thieno[3,2-*d*]pyrimidin-7-yl)phenyl)propanoic acid;
62) 3-(4-(4-(4-(3-(2-fluoro-3-(trifluoromethyl)phenyl)ureido)phenyl) thieno[3,2-*d*]pyrimidin-7-yl)phenyl)propanoic acid;
63) 3-(4-(4-(4-(3-(4-fluoro-2-(trifluoromethyl)phenyl)ureido)phenyl) thieno[3,2-*d*]pyrimidin-7-yl)phenyl)propanoic acid;
64) 3-(4-(4-(4-(3-(3-chlorophenyl)ureido)phenyl)thieno[3,2-*d*]pyrimidin-7-yl)-2-fluorophenyl)propanoic acid; and
65) 1-(3-chlorophenyl)-3-(4-(7-(4-(methylsulfon)phenyl)thieno[3,2-*d*]pyrimidin-4-yl)phenyl)urea.

4. A method for preparing the compound of formula (I) of claim 1, which comprises:
(a) subjecting 7-bromo-4-chlorothieno[3,2-*d*]pyrimidine or N-substituted 7-bromo-4-chloro-pyrrolo[3,2-*d*]pyrimidine to a Suzuki coupling reaction with *tert*-butyl 4-(4,4,5,5-tetramethyl-1,3,2-dioxaborolan-2-yl)phenylcarbamate to obtain the compound of formula (II) by replacing the chloride in position 4 of thieno[3,2-*d*]pyrimidine or pyrrolo[3,2-*d*]pyrimidine, repeating the Suzuki coupling reaction once again, followed by a deprotection reaction; and 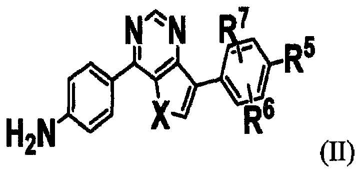 wherein X, R⁵, R⁶ and R⁷ are the same as defined in the compound of formula (I) of claim 1,
(b) allowing the compound of formula (II) to react with isocyanate or thioisocyanate to obtain the compound of formula (I); or alternatively subjecting an aniline compound to react with substituted phenyl chloroformate or substituted phenyl thiochloroformate to form a compound, and then subjecting the compound thus obtained to react with the compound of formula (II) to obtain the compound of formula (I).

5. A pharmaceutical composition for inhibiting the activity of diacylglycerol O-acyltransferase type 1 (DGAT1) comprising the compound of claim 1 as an active ingredient.

6. The pharmaceutical composition of claim 5, which is used for the prevention or treatment of a disease or condition mediated by the activity of DGAT1

7. The pharmaceutical composition of claim 6, wherein the disease or condition is selected from the group consisting of obesity, hyperlipidemia, hypertriglyceridemia, lipid metabolism disorders, insulin resistance syndrome, glucose intolerance, diabetes, diabetic complications, cataract, gestational diabetes, non-alcoholic fatty liver disease, polycystic ovary syndrome, arteriosclerosis, atherosclerosis, diabetic sclerosis, ischemic heart diseases, bulimia, hypertension, cerebrovascular disorders, coronary artery disease, fatty liver, respiratory abnormality, backache, gonarthrosis, gout and cholelithiasis.
